# EUROPEAN PATENT SPECIFICATION

(11) **EP 2 501 493 B1**
(45) Date of publication and mention of the grant of the patent: **22.08.2018**
(21) Application number: 10782353.6
(22) Date of filing: 16.11.2010
(51) Int. Cl.: E03D 9/00, B05B 17/06, A01M 1/20, A61L 2/22, A61L 9/14

(54) **LAVATORY TREATMENT DEVICE AND METHOD**
VORRICHTUNG UND VERFAHREN ZUR TOILETTENBEHANDLUNG
DISPOSITIF ET PROCÉDÉ DE TRAITEMENT DE TOILETTES

(30) Priority: 18.11.2009 US 262393 P
(43) Date of publication of application: 26.09.2012
(73) Proprietor: Reckitt Benckiser LLC, Parsippany, NJ 07054 (US)
(72) Inventor: BURT, Diane, Joyce, New Windsor, New York 12553 (US); CREEK, John, Aubrey, Bridgewater, New Jersey 08807 (US); HINDLE, Benjamin, David, Ridgewood, New Jersey 07540 (US)
(74) Representative: Cawdell, Karen Teresa
(86) International application number: PCT/GB2010/002097
(87) International publication number: WO 2011/061478

(56) References cited:
- WO-A1-2009/090909
- GB-A- 2 405 097
- US-A1- 2007 235 555
- US-A1- 2008 223 953

## Description

The present invention relates to devices directed to devices and methods for delivering treatment compositions to a lavatory appliance, especially a toilet bowl.

Chemical compositions for providing a technical benefits to surfaces are notoriously old and known to the art. Liquid compositions, which are frequently largely aqueous in their composition, may be supplied to a surface by any of a number of means including simply pouring a quantity of such a composition of the surface, or delivering it as a liquid stream from a nozzled container, or delivering it in the form of droplets which are delivered from a dispensing container. Widely used dispensing containers include pressurized container such as aerosol canisters which include a quantity of the composition as well as a propellant, as well as nonpressurized flasks or vessels which are equipped with a manually-pumpable spray head which can be used to dispense the compositions via a nozzle. While such are effective in many circumstances, they are not without disadvantages. Typically, the delivery rate using an aerosol canister or a manually-pumpable spray head is effective, but the relatively large droplets delivered by such means typically quickly saturate a hard or soft surface upon which they are dispensed. Further, the relatively large individual droplets delivered by such means are also often of a wide range of particle size masses or diameters does providing a very low degree of uniformity with regard to the dimension of the average drop or particle size and being delivered. While such as advantageous where a large quantity of such treatment composition is intended to be relatively quickly delivered or deposited onto a surface, such is also disadvantageous as the relatively large drop the particle size quickly drops to the surface and provides a limited degree of distribution of the treatment composition a hard surface. The use of bottles or flasks containing and delivering a fluid or liquid treatment composition to a hard surface is also well known. Typically such bottle or flask include a nozzle at the open end of the bottle or flask, which is tilted, inverted or compressed to cause the treatment composition to flow in exit via the nozzle. The nozzle is directed proximate to a surface being treated, and thus a good degree of control of the application of the composition is achieved. However, typically a relatively large volume or mass of the treatment composition is dispensed in such manners, and is required in order to ensure good surface coverage of the surface being treated. This particularly true where inclined surfaces, or curved surfaces, e.g., the interior of a lavatory appliances such as toilet bowls, urinals, bidets and the like are intended to be treated with the composition. The application of excess amounts of such treatment composition in such a manner can be seen to be wasteful, and uneconomical. Thus, there is a real need in the art for providing improved methods for the delivery of treatment compositions lavatory surfaces. It is to such a need that the present invention is directed. US2008/223953, WO2009/090909 and US2007/235555 dicloses a device for the treatment of a lavatory appliance, which device generates a mist.

Also generally known to the technical arts primarily directed to air treatment, e.g., dispersion of fragrances, perfumes, insecticides, air fresheners, odor neutralizers, into an airspace are various devices for dispensing a liquid composition in the form of dispersed particles. Such include those disclosed in US 7694892 to Feriani, et al.; US 2009/308945 to Tollens, et al.; US 2009/272818 to Valpey III, et al.; US 5299739 to Takahashi et al.; which disclose various devices which include a vibrating plate and a wick or capillary for delivery of liquids from a reservoir to the vibrating plate. Further, US 2004/0256487 to Collins, Jr. et al., and US 2005/0103891 to Abergel, et al. disclose spraying devices which include a vibrating plate in direct fluid contact with liquid from a reservoir. US 5297734 discloses various arrangement of vibrating plates supplied with liquids for delivering particulates of the liquid to an airspace.

Notwithstanding these known art devices, further advances are still needed in the art treatment devices and treatment methods.

In one aspect of the present invention provides a device for the treatment of a lavatory appliance which device generates a mist of a treatment composition which imparts a technical benefit to surfaces of the lavatory appliance which come into contact with the said aerosolized treatment composition, the device comprising: a mist generator means including an atomizing chamber, a control circuit for operating the mist generator means, a reservoir for the fluid product to be aerosolized, a means for supplying the mist generating means with the fluid product, a housing, and at least one flow directing nozzle, flow directing implement or flow directing orifice adapted to direct the flow of a mist generated by the mist generating means out from the housing and towards part of a lavatory appliance, wherein that the atomizing chamber has a base, a generally perpendicular side wall which terminates at a top, the top extends from the side wall to an inner sidewall which is generally perpendicular to the top and extends downwardly or inwardly towards the base therefrom, until the inner sidewall terminates at an inner sidewall base, characterised in that the mist has a bi-modal distribution of fine particles, with a first portion having a particle size of 1-8 microns and a second portion having a particle size of 10-40 microns.

In a still further aspect of the invention there is provided a method for the delivery of an air treatment composition to an airspace in the proximity of a lavatory appliance, which method comprises the step of: providing a device which generates a mist of a treatment composition, which treatment composition contacts said airspace within the proximity of the lavatory appliance and provides a technical benefit thereto.

The mist generator means may comprise a vibrating member which includes a metal or ceramic plate; the plate may be solid or porous, or micropierced in the form of a grid and/or in the form of one or more segments or slots passing through the vibrating member, and a piezoelectric actuator which, when operated, causes vibratory motion in the vibrating member. The mist generator means may be an annular ring of a piezoelectric material which is attached to said vibrating member and spans the annulus, which when activated, causes the said vibrating member to vibrate. The mist generator means may comprise a piezoelectric material which is attached to, adjacent to or abutting a non-vibrating element or member which receives the vibratory motion of the piezoelectric material and transfers the vibratory motion to the said vibrating member. The mist generator means may be a tubular piezoelectric material which includes a vibrating member spanning its interior bore between the ends of the piezoelectric material, and/or includes a vibrating member spanning the interior bore at one or more ends thereof, such that when activated the tubular piezoelectric material vibrates or expands/contracts which in turn imparts vibratory motion of the vibrating member(s).

The mist generator means may be an electrostatic spray device. The mist generator means may be an ultrasonic nozzle device.

The mist generator means may be a tubular aerosol generator which includes a tube having a first and a second end, a heater arranged relative to the tube for heating the tube, a source of material to be volatilized, the second end of the tube being in communication with the source of material, a valve operatively located between the source of material and the tube, the valve being openable and closeable to open and close communication between the source of material and the first end of the tube, and a pressurization arrangement for causing material in the source of material to be introduced into the tube from the source of material when the valve is in an open position.

The mist generator means may form a part of the device and be permanently affixed thereto. Alternately the mist generator means may be provided as part of a refill unit or refill reservoir which, when inserted or affixed to the device completes the device and enables its use. Further the mist generator means may be user replaceable article or unit which may be removed and/or installed as needed or desired by a user to one or more of the device or the refill unit or refill reservoir. Yet further in any embodiment, the mist generator may be formed of several parts which are required to be assembled in order to form an operating mist generator means, e.g., a piezoelectric actuator may form part of the device and a separate vibrating member form part of a refill unit or refill reservoir which remains inoperative until the device and refill unit or refill reservoir are properly aligned or otherwise installed in the device so permit the interaction between the piezoelectric actuator and the vibrating member which then operates as a mist generator means. Such an embodiment is preferred in that with the replacement of a refill unit or refill reservoir a new vibrating member is provided to the device.

The device includes a controller means for controlling the operation of the mist generator means. The controller means may provide one or more functions. The controller means preferably includes a high frequency generator used to generate a suitable electrical signal for the operation of the mist generator, and particularly a piezoelectric element or device associated therewith. The controller means may include one or more switches, or other input means, e.g., buttons, contacts or switches, which can be established by user of a device according to the invention in order to control the mode of operation of the controller means. The controller means may also include means for controlling the output of the mist generator which may turn the unit off, or suspend its operation after a metered amount or dose of the treatment composition is dispensed from the device; the amount of the treatment composition may be a user controllable amount, e.g., via a setting, or may be a predetermined metered amount which cannot be changed by the user. The amount of treatment composition delivered by the device may be varied in response to a signal received by the controller means which may respond to an environmental condition of the device. The controller means may also be adapted to receive, and respond to, one or more signal inputs received from one or more sensors associated with the device. For example the controller means may be adapted to receive and respond to signals or conditions relating to the status of any part of the device such as the quantity of treatment composition in a reservoir or refill unit, to the physical orientation of the device, as well as to the frequency of dispensing and/or volume of treatment composition dispensed over a unit time interval. Nonlimiting examples of such responses include to increase or decrease one more of: the volumetric delivery rate of the treatment composition, and/or the frequency of delivery of the treatment composition per unit of time. The controller means may provide one or more output signals which may be transmitted to one or more further elements of the device via suitable conductor means, such as wires, in order to control their operation. The controller means may be programmable and include suitable electronic circuitry for the operation of the device according to one or more programs each having at least one, but preferably a plurality of, discrete programmed steps; typically such includes at least a logic or program controller, e.g., a central processing unit, and system memory for storing one or more programs. The controller means may be a non-programmable circuit, which preferably operates according to specific logic responsive to one or more signal inputs to the controller means. The controller means may comprise a drive circuit in order to provide suitable power and/or signal outputs to the mist generator in order to control its operation in generating a treatment mist from the fluid treatment composition, which may include known-art drive circuits suitable for this purpose. One example of a suitable circuit which may be present within the controller means is a pulse-width-modulation circuit (PWM) comprising a transformer converter and having an input acted on by a piezoelectric element present and the mist generator; such as disclosed in published application US 2009/0121043. A further example of a suitable circuit is one which includes a microprocessor controlled variable oscillator for providing variable frequencies to mist generator such that treatment composition is formed into an aerosol of fine droplets. The variable oscillator preferably comprises a digital resistor for adjusting the time of charge and discharge; such a circuit is disclosed in US 7673812.

The device may be operated by direct control by a user, e.g., controlling a switch upon the device or alternately, the device may be operated indirectly, e.g., by a remote control unit.

The device may include a power supply source which is integral to the device, e.g., one or more batteries, such that the device is portable, or the device may include means, e.g. wire, for connecting the device to a source of power, e.g., a transformer or electrical mains, supplying electrical power to the controller means.

The device may include one or more sensor means. Sensor means may be present to evaluate the state of a condition within the device, e.g., the presence of a treatment composition, or the presence of a suitable refill container. Sensor means may be present to evaluate the state of the environment in which the device is being used, e.g., time of day, degree of brightness near the environment of the device, absence of light, presence of light, a sound sensor, a vibration sensor, a heat sensor, an odor or scent sensor, a pressure sensor, a proximity sensor, and the like.

The device may operate autonomously, e.g., be fully controlled by the controller means when the device is operative. The device may operate semi-autonomously, e.g., be operative through the periodic interaction with a user, such as by sensing the user and/or by user interaction and operation with the controller means, such as by operating one or more switches, or other input means.

The device may include a fill level sensor which controls the operation of the device responsive to the amount of liquid present in the device and/or in the reservoir, which may be a removable reservoir.

The device may include one or more orientation sensing means for determining a physical orientation of the device, which for example, can be a level sensor, horizon sensor, accelerometer or any other device which can be used to establish the relative position of the device with respect to the horizontal or horizon.

The device may include a reservoir for containing a quantity of the treatment composition, which reservoir may be a integrally formed as part of, or as an element of the device, which is not intended to be removed but rather refilled with the treatment composition when required. Alternately the device may include a removable reservoir which is intended to be removed from the device and replaced when necessary, such as to replenish or to resupply a new quantity of the treatment composition to the device. The reservoir of the device may be adapted to contain a single fluid treatment composition or may be adapted to contain a plurality of fluid treatment compositions.

The device may include at least one fluid control means for controlling the rate of delivery of a fluid product, viz., a treatment composition, from the reservoir to the mist generator. The fluid control means may form part of the device, or may be part of a removable reservoir, or may be present in both the device and a removable reservoir. The fluid control means may also be formed by cooperative elements, part of which are present on the removable reservoir and part on the device such that, when the cooperative elements are assembled, in conjunction they form a fluid control means. The device may include one, or several fluid control means. Nonlimiting examples of fluid control means include the following: a) one or more capillaries which via capillary effect supply the treatment composition from the fluid reservoir to the mist generator means; b) one or more tubes or channels which provide fluid conduits to supply the treatment composition from the fluid reservoir to the mist generator means; c) one or more pumps, d) direct physical interaction between a vibrating member and the treatment composition, e.g. wherein the treatment composition is supplied to a top surface or bottom surface of the vibrating member during at least a portion of its range of vibratory (or oscillatory) movement, or during the range of vibratory (or oscillatory movement) the vibrating member contacts a quantity of the treatment composition and entrains it within the vibrating member before expelling it therefrom, such for example may occur wherein a wick or a tube having exposed treatment composition at an end thereof is in near proximity but not in direct contact with a vibrating member; e) by a gravity feed flow of the treatment composition to the mist generator means; f) a manual supply means, e.g., manual pumping by a user of an element such as a pump or bulb which transfers a quantity of the treatment liquid to the mist generator means; g) an antechamber or cavity which is intermediate the reservoir and the mist generator means which antechamber or cavity is first filled from the reservoir, and the mist generator means is supplied with treatment composition from the antechamber of cavity but not directly from the reservoir.

Particularly preferred fluid flow means include c) one or more pumps, including but not limited to: gear pumps, positive displacement pumps, rotary pumps, micropumps, diaphragm pumps, and especially preferably piezoelectric pumps such as those presently commercially available from Bartels Mikrotechnik GmbH (Dortmund, Germany). Examples of such piezoelectric pumps are disclosed in one or more of the following: WO/2009/059664. Such are particularly preferred embodiments of the invention.

The device may include an airflow generator means. The airflow generator means may be used to generate a current of air which induces or directs the flow of the atomized treatment composition, and especially as it exits the device. The airflow generator means also entrains the nebulized or mist of the treatment composition and may be used to direct its flow outwardly from the device.

The device may be a single unit which is substantially confined by a housing, or maybe comprised of a plurality of elements which cooperatively operate to provide a device according to the invention.

The device may comprise further flow directing elements or distribution implements which provide for controlling the distribution of the mist of the treatment composition to provide improved delivery of the same to the surfaces being treated.

In certain preferred embodiments, the device includes attachment means for removably attaching the device, or parts of the device to a lavatory appliance. Single, multiple attachment means may be present depending on the configuration of the device.

While the device of the invention is moveable, and may be installed or mounted upon or in the proximity of a lavatory appliance or other locations with a lavatory or bathroom, preferably the device is in a stationary position or location during dispensing of the mist of the treatment composition.

The device may further comprise an air-treatment means which is used to provide a volatile material to the ambient environment of the device, which volatile material is supplied to the ambient environment independently of the mist generator means. The air-treatment means may be used to deliver a volatile material, e.g., one or more of fragrances, perfumes, compositions for the control or eradication of airborne insects, odor neutralizing agents, odor masking agents, as well as those which may impart holistic or aromatherapy benefits which is separate from the treatment composition. For example, such a volatile material may be provided in a reservoir comprising a quantity of said volatile material which may form part of or be used with the device. Such a reservoir can take any shape or suitable form, and can be included within the interior of the device, or on the exterior of the device, or may be even be separate from the device but provided as a separate article or element which is separate or separable from the device but intended to be placed in the near proximity of the device. By way of nonlimiting examples, such a reservoir may include a porous material such as a pad or tablet which is impregnated with, or upon which is absorbed a volatile composition useful in providing an air treatment benefit, a gel or a solid composition which also contains a volatile air treatment composition which may emanate to the ambient environment from the reservoir, or a container which includes a fibrous wick, or pad, or a porous membrane for the delivery of a volatile material to the ambient environment from the reservoir. Alternately the reservoir may contain a quantity of a particulate material in the form of a single body, e.g. plate, or as a plurality of spheres, or beads which function as a reservoir for the volatile composition, and from whence they may be delivered to the ambient environment. Non-limiting examples of such materials include those currently marketed under the tradename Auracell® (ex. Rotuba Extruders) which are based on fragranced cellulosic polymers, as well as PolylFF® (ex. International Flavors and Fragrances Inc.), as well as Tenite® (ex. Eastman Chemical Co.).

The device of the invention includes a mist generator means for the delivery of a treatment composition which comprises a treatment agent. In certain embodiments the treatment composition may be solely comprised of the treatment agent. The mist generator may be any device which provides for atomization of the treatment composition or which provides for the aerosolization of the treatment composition without directly heating the treatment composition or utilizing a propellant gas or the use of a liquid pump to drive the treatment composition through a nozzle and consequently cause the formation of discrete particles therefrom.

In preferred embodiments the mist generator means is a nebulizer means, which is also generally preferred for use. Nebulizer sprayers impart energy to the treatment composition. The ultrasonic energy supplied via a transducer. This energy results in atomization of the treatment composition without requiring direct heating of the treatment composition or without the need for a propellant composition, or a manually operated liquid pump to drive the treatment composition through a nozzle to aerosolize the treatment composition. Various types of nebulizers include, but are not limited to: ultrasonic, gas, venturi, and refillable nebulizers. Such may be obtained from a variety of commercial sources.

Exemplary and preferred embodiments of a nebulizer means which are presently commercially available from Kai-Chih Industrial Ltd. (Taiwan) are disclosed in one or more of US 6854662; a nebulizer and baffle plate assembly as disclosed in US 7229029; piezoelectric and percussion board assembly as disclosed in US 2007/0011940; a block piezoelectric actuator and vibratable plate as disclosed in US 2007/0169775; a vibration member comprising a piezoelectric ceramic actuator and a vibratory plate as disclosed in US 2008/00419272.

A "bi-modal" distribution of very fine liquid particles are provided by a nebulizer, such that, opposed to many known nebulizers which provide a distribution of very fine liquid particles which are averaged about a median or averaged liquid particle size or liquid particle mass, in said preferred embodiment the nebulizer provides a bi-modal distribution of very fine liquid particles, a first part or proportion of the liquid particles being of a first particle size distribution which are averaged about a first median or first averaged liquid particle size or liquid particle mass, and a second part or proportion of the liquid particles being of a second particle size distribution which are averaged about a second median or second averaged liquid particle size or liquid particle mass. In such embodiments, the average liquid particle size or liquid particle mass of the first median or first particle size distribution is lesser in average or median particle size or mass than the average liquid particle size or liquid particle mass of the second median or second particle size distribution. The provision of such a bi-modal distribution provides for a first part or portion of the liquid particles being of a smaller particle size, preferably having a first median or first averaged liquid particle size in the range of 1 - 8 microns, yet more preferably between 2 - 7 microns, and a second part or portion of the liquid particles being of a relatively larger particle size, preferably having a second median or second averaged liquid particle size in the range of 10 - 40 microns, yet more preferably between 10 - 35 microns. Optionally but advantageously, at least 60%, and in order of increasing preference, at least 70%, 75%, 80%, of the particles or mass of the liquid particles present within the first or second proportion are within +/- 35% by mass or size, and in order of increasing preference are within" +/- 30%, +/- 25%, +/- 20%, +/- 15%, +/-10% of their respective median or average liquid particle size or liquid particle mass. Such provides for a narrowed distribution of the liquid particle sizes or masses delivered by the nebulizer. Further preferably, the mass of the particles delivered in the first part or portion of liquid particles is not more than about ½, preferably not more than about 1/4 of the mass of the mass of the particles delivered in the second part or portion of liquid particles, which have a larger average particle size or mass. Alternately, but preferably, the mass ratio of the particles delivered in the first part or portion of liquid particles to the particles delivered in the second part or portion of liquid particles is in the range of about 1:2, and in order of increasing preference is in the respective mass ratio about: 1:3, 1:4, 1:5, 1:6, 1:7, 1:8, 1:9, 1:10. The delivery of the liquid treatment composition as a bi-modal distribution of very fine liquid particles provides for controlled distribution of the treatment composition wherein a controlled mass, but visually very visible amount or mass, of the liquid treatment composition provided in the a first part or proportion of the liquid particles is delivered concurrently with greater mass of the liquid treatment composition provided in the a second part or proportion of the liquid particles. Such minimizes or reduces the amount of treatment composition which is delivered as smaller, potentially respirable liquid droplets or particles which are nonetheless airborne and more buoyant than the greater mass of the treatment composition which is delivered as larger, less potentially respirable liquid droplets or particles of the treatment composition.

Mists of the treatment composition, interchangeably referred to as a treatment mist, have several advantages. A first advantage is that it is flowing and somewhat buoyant, which permits for the deposition of the very fine liquid particles on surfaces which are not necessary adjacent to the outlet of a device from whence the mist is released. This may provide for a small degree of airborne drift and permit for improved deposition of the liquid particles as compared to liquids which may be applied via a manually pumped trigger sprayer, or even liquids dispensed from a pressurized aerosol container. In the case of the former, the droplets of a liquid composition dispensed from a manually pumped trigger sprayer typically have larger average droplet sizes than those delivered by such a mist generator, and hence average droplet masses which concurrently transport and bombard a treated surface with greater amounts of a liquid composition per droplet. Such characteristics minimize the aerial buoyancy of the droplets, and when the droplets contact a surface the greater mass of liquid composition tends to much more quickly wet a surface, primarily by adsorption and to a lesser extent by absorption. Thus, both the larger and heaver particle sizes of the such liquid droplets, and their velocity as they are being released from a manually pumped trigger sprayer typically causes greater amounts of a liquid composition to be dispensed and faster wetting of surfaces. Turning to the latter, delivery of a liquid composition from a pressurized aerosol container typically results in similar delivery characteristics. While a critical selection of the orifice sizes and internal passages of an aerosol canister's spray actuator typically used with such pressurized aerosol container often provides somewhat more selection and control of the average droplet size, still the typical droplets of a liquid composition dispensed from a pressurized aerosol container also typically have larger average particle sizes than those delivered by such a generator, and hence have average droplet masses and greater distribution of average droplet sizes which concurrently transport and bombard a treated surface with greater amounts of a liquid composition per droplet. Such characteristics minimize the aerial buoyancy of the droplets, and when the droplets contact a surface the greater mass of liquid composition tends to much more quickly wet a surface, primarily by adsorption and to a lesser extent by absorption. Further, as the droplets dispensed from a pressurized aerosol container are typically released at a higher linear velocity than even the droplets released from a manually pumped trigger sprayer, such even moreso diminishes the likelihood of aerial buoyancy and airborne drift.

The delivery rates of the devices may vary in order to suit a specific application, e.g., it may be advantageously to have a higher delivery rate of the treatment composition per unit of time (e.g., seconds, minutes, hours, days) for spaces with larger volumes and/or wherein the device is located at a greater distance from the surface or surfaces to be treated, as opposed to closer placement and/or smaller volumes or spaced to be treated. Advantageously the treatment mist dispensed from the device may be delivered at a rate of about 0.5 milliliter/minute to about 100 milliliter/ for most applications and uses. Preferably the delivery rate is from about 1 - 50, more preferably 1 - 25, still more preferably 1 - 10 and particularly preferably about 1 - 5 milliliter/minute.

Optionally but preferably the treatment mist emitted from the mist generator in devices according to the invention may travel along a horizontal surface for a reasonable distance when exiting the device. Preferably the plume of the treatment mist emitted from the mist generator travels up to 60 cm in a lateral or horizontal direction perpendicular to the device, and preferably travels between 1 - 50 cm in such a direction as measured from where it exits the device. Such permits for the travel, distribution and contact of the treatment mist with surfaces having non-planar geometries, e.g., the interiors of devices, e.g. the interior surfaces of a lavatory appliance such as toilet bowl.

The treatment mist emitted from the mist generator in devices according to the invention may provide improved and more uniform deposition onto hard or non-porous surfaces, particularly when such may be associated with articles having three dimensional features, or which themselves have a three-dimensional, e.g., patterned, non-flat planar, or roughened surface or wherein the surfaces are other than flat, planar and horizontal, e.g., are vertical, inclined or curved surfaces. The geometry or irregularities in such surfaces may be very effectively treated by providing a mist from a device according to the invention, or according to a process of the invention in the near proximity or adjacent to such a surface, such that the delivered mist is permitted to settle and deposit upon such a three-dimensional surface. The delivery of the mist, which is expected to be airborne for at least several seconds after being dispensed from a device, will often also exhibit a useful degree of airborne suspension and drifting prior to settling upon a hard surface to being treated therewith. Such airborne drifting provides for improved coverage of hard surfaces, particularly when such are three-dimensional themselves or are associated with articles having three dimensional features. Such include the surfaces of a lavatory appliance, e.g. a toilet, bidet, shower, bathtub, or bathroom sink which defining closures which can be used to retain the buoyant mist of the treatment composition delivered by the device. The delivery of a treatment composition in the form of an airborne mist of the treatment composition, which may be alternately characterized as a cloud of very fine liquid particles of the treatment composition provides for improved surface deposition on such surfaces, including that of such elements. Due to the airborne nature of this mist or cloud, the dispensed mist or cloud forms an enveloping body or penumbra of very fine liquid particles of the treatment composition which may first surround a surface or article, and then deposit thereon by settling of the very fine liquid particles. Furthermore, the delivery of a treatment composition in the form of an airborne mist of the treatment composition which can be suspended within an interior cavity, space, or volume of a lavatory appliance, particularly the bowl of a toilet appliance, the bowl of a bidet, the interior of a urinal, the interior of a bathtub, the interior of a shower stall, the base or pan of a shower stall, as well as the interior of a lavatory sink or wash basin can be advantageously provided with the mist of the treatment composition by the use of a device according to the invention. The treatment composition may be applied to any surface of the lavatory appliance, e.g., may be applied to the interior surfaces of a toilet bowl, the rim of a toilet bowl, beneath the rim of a toilet bowl, the underside of the seat of a toilet bowl, and preferably such treatment of two or more of the foregoing surfaces is effectuated essentially concurrently or simultaneously and does not require user intervention, e.g., scrubbing, wiping, and the like to distribute the treatment composition onto the treated surface(s). The delivery of a mist or cloud of the very fine liquid particles of the treatment composition generated by the device which he subsequently at least for a short time, suspended within such an interior cavity, space or volume of a lavatory appliance, e.g., toilet bowl, allows for a generally uniform distribution within such interior cavity, space or volume as it is airborne, and provides a generally uniform distribution of the very fine particles forming the mist as they settle downwardly onto the surface is of the interior cavity, space or volume. Such permits for a very effective delivery of the quantity of the treatment composition via its mist form, or providing a surface coating or surface lamina upon the treated hard surface, which typically is ample in order to provide a desired technical benefits thereto.

A further important technical characteristic of the delivery of a treatment composition as an airborne mist of the treatment composition is that typically better surface coverage and a more uniform layer of a treatment composition is deposited on the surface of a lavatory appliance or other lavatory surface, and thus the actual mass of a treatment composition may be reduced as compared to delivery of the same treatment composition via a nozzled flask or bottle, a manually pumped trigger sprayer or a pressurized aerosol container in order to achieve a comparable technical effect. More simply stated, less of the treatment composition is wasted due to excessive delivery or overspraying than when delivered as a mist or cloud of very fine liquid particles of the treatment composition. Such is beneficial when for example, the delivery of a treatment composition providing a surface cleaning, sanitizing or antimicrobial benefit is desired, or where a film forming polymer is intended to be applied to a hard surface. As a more uniform deposition of the treatment composition may be achieved. Such reduces the uneconomical usage of greater amounts or masses of a fluid or liquid form of the treatment composition as typically occurs when delivering such from a nozzled flask or bottle, while achieving the same or comparable technical benefits.

The device of the invention generates a treatment mist of discrete or aerosolized particles of the treatment composition which is used to treat surfaces, including inanimate hard surfaces, especially preferably lavatory surfaces and particularly preferably the surface of lavatory appliances. The aerosolized form of a treatment composition comprises at least one treatment agent which ultimately contacts a surface being treated after being dispensed from the device of the invention. The treatment agent may be provided as a constituent of a treatment composition comprising further constituents other than the treatment agent, although a treatment composition consisting solely of a treatment agent is not excluded from the scope of the invention.

The treatment composition comprises at least one treatment agent. The treatment composition provides a technical benefit to a hard surface or soft surface being treated. By way of nonlimiting examples, such a technical benefit can be one or more of: a cleaning benefit, a disinfecting benefit, a sanitizing benefit, an anti-allergen benefit, an anti-acaricidal benefit, an anti-fungal benefit, an anti-resoiling benefit, a limescale removing benefit, a stain removing benefit, an air treatment benefit including but not limited to; fragrancing, odor masking, odor neutralization, an anti-pesticidal benefit, an anti-insecticidal benefit, as well as providing a surface coating to hard surfaces. The treatment composition as applied to hard surfaces and/or soft surfaces may provide a technical benefit which may be transitory or durable, e.g., provide a residual antimicrobial, germicidal or sanitizing benefit such as to reduce the likelihood of the retention, or growth of undesired pathogens (e.g., bacteria, virus, molds) on the treated surface. The treatment composition may also reduce the buildup of biofilms on the treated surface, may reduce the incidence of limescale and/or its buildup after being treated. The treatment composition may provide a surface shine benefit to treated surfaces. The treatment composition may provide an antiresoiling benefit. The treatment composition may deposit a coating on hard surface or soft surface which is hydrophilic in nature or hydrophobic in nature. Treatment compositions which are formed into treatment mists necessarily comprise an effective amount of one or more treatment agent within the treatment composition such that the desired technical benefit is provided when the treatment mist is applied to or into a lavatory appliance.

Prior to being formed into a treatment mist, the treatment composition is advantageously a flowable liquid at room temperature (20°C) and at normal atmospheric pressure in which the device of the invention finds use. The viscosity of the treatment composition is not necessarily critical, it only being required that it can be atomized in the device out of the invention and delivered as a mist of comminuted or aerosolized particles. Advantageously however the viscosity of the treatment composition falls within the range of about 0 - 2000 cP, preferably between about 0.5 - 1000 cP, and especially preferably between about 0.5 - 500 cP. Especially preferred embodiments of the treatment composition are free flowable liquids, i.e. are "water thin" and thus are readily flowable, as well as being readily pump a bowl either by mechanical means such as by a pump, or by pressure different means such as within a capillary or narrow diameter tube, and which is also readily easily and effectively atomized by the mist generator means.

Advantageously, the treatment composition includes a large proportion, that is to say at least about 50%wt. of a liquid. The liquid is preferably a free-flowing liquid at room temperature and normal prevailing atmospheric conditions as noted above. Advantageously, the liquid may be water, maybe one or more organic solvents, or may be a mixture or composition comprising both water and one or more organic solvents. The water may be tap water, but is preferably distilled and is most preferably deionized water. By way of non-limiting example exemplary useful organic solvents which may be included in the treatment compositions include those which are at least partially water-miscible such as alcohols (e.g., low molecular weight alcohols, such as, for example, ethanol, propanol, isopropanol, and the like), glycols (such as, for example, ethylene glycol, propylene glycol, hexylene glycol, and the like), water-miscible ethers (e.g. diethylene glycol diethylether, diethylene glycol dimethylether, propylene glycol dimethylether), water-miscible glycol ether (e.g. propylene glycol monomethylether, propylene glycol mono ethylether, propylene glycol monopropylether, propylene glycol monobutylether, ethylene glycol monobutylether, dipropylene glycol monomethylether, diethyleneglycol monobutylether), lower esters of monoalkylethers of ethylene glycol or propylene glycol (e.g. propylene glycol monomethyl ether acetate), and mixtures thereof. Glycol ethers having the general structure Rₐ-R_{b}-OH, wherein Rₐ is an alkoxy of 1 to 20 carbon atoms, or aryloxy of at least 6 carbon atoms, and R_{b} is an ether condensate of propylene glycol and/or ethylene glycol having from one to ten glycol monomer units. Of course, mixtures of two or more organic solvents may be used concurrently.

One preferred organic solvent which may be included within the treatment compositions is triethylene glycol which is believed to provide odor sanitization or odor neutralizing benefits to an airspace within which culminated particles of triethylene glycol are present. Thus come in certain embodiments were such a technical benefit is desired, the inclusion of triethylene glycol may be considered for its advantageous benefit. When present, it can be included in amounts effective to provide a desired degree of air sanitization.

The treatment composition may also include one or more surfactants. The presence of one or more such surfactants which are advantageously included to typically provide for the loosening of soils or other hydrophobic matter which may be present on a surface being treated with the device of the invention.

Anionic surfactants and/or salt forms thereof may form part of the inventive compositions. Non-limiting examples of anionic surfactants include alcohol sulfates and sulfonates, alcohol phosphates and phosphonates, alkyl ester sulfates, alkyl diphenyl ether sulfonates, alkyl sulfates, alkyl ether sulfates, sulfate esters of an alkylphenoxy polyoxyethylene ethanol, alkyl monoglyceride sulfates, alkyl sulfonates, alkyl ether sulfates, alpha-olefin sulfonates, beta-alkoxy alkane sulfonates, alkyl ether sulfonates, ethoxylated alkyl sulfonates, alkylaryl sulfonates, alkylaryl sulfates, alkyl monoglyceride sulfonates, alkyl carboxylates, alkyl ether carboxylates, alkyl alkoxy carboxylates having 1 to 5 moles of ethylene oxide, alkylpolyglycolethersulfates (containing up to 10 moles of ethylene oxide), sulfosuccinates, octoxynol or nonoxynol phosphates, taurates, fatty taurides, fatty acid amide polyoxyethylene sulfates, acyl glycerol sulfonates, fatty oleyl glycerol sulfates, alkyl phenol ethylene oxide ether sulfates, paraffin sulfonates, alkyl phosphates, isethionates, N-acyl taurates, alkyl succinamates and sulfosuccinates, alkylpolysaccharide sulfates, alkylpolyglucoside sulfates, alkyl polyethoxy carboxylates, and sarcosinates or mixtures thereof. Anionic soaps may also be used in the inventive compositions. Examples of the foregoing anionic surfactants are available under the following tradenames: Rhodapon®, Stepanol®, Hostapur®, Surfine®, Sandopan®, and Biosoft® tradenames.

Exemplary useful nonionic surfactants are those which include a hydrophobic base portion, such as a long chain alkyl group or an alkylated aryl group, and a hydrophilic chain portion comprising a sufficient number of ethoxy and/or propoxy moieties to render the nonionic surfactant at least partially soluble or dispersible in water. By way of non-limiting example, such nonionic surfactants include ethoxylated alkylphenols, ethoxylated and propoxylated fatty alcohols, polyethylene glycol ethers of methyl glucose, polyethylene glycol ethers of sorbitol, ethylene oxidepropylene oxide block copolymers, ethoxylated esters of fatty (C₆-C₂₄) acids, condensation products of ethylene oxide with long chain amines or amides, and mixtures thereof. Further useful nonionic surfactants include condensates of alkylene oxides, particularly ethylene oxide with sorbitan fatty acid esters, e.g., polyoxyethylene sorbitan monolaurate, polyoxyethylene sorbitan monopalmitate, and polyoxyethylene sorbitan trioleates. Still further useful nonionic surfactants include alkoxylated alkanolamides, e.g. C₈-C₂₄ alkyl di(C₂-C₃ alkanol amide). Examples of the useful nonionic surfactants include materials are available under the Tomadol®, Neodol®, Rhodasurf®, Genapol®, Pluronic® and Alfonic® tradenames. Further useful nonionic surfactants include oxo-alcohol ethoxylates (ex. BASF) under the Lutensol® ON tradename, as well as polyoxyalkylene alkylethers (ex. KAO Group, Japan) available under the Emulgen® tradename.
A further useful nonionic surfactants include alkylmonoglycosides and alkylpolyglycosides are prepared generally by reacting a monosaccharide, or a compound hydrolyzable to a monosaccharide with an alcohol such as a fatty alcohol in an acid medium. Various glycoside and polyglycoside compounds including alkoxylated glycosides and processes for making them are disclosed in U.S. Pat. Nos. 2,974,134; 3,219,656; 3,598,865; 3,640,998; 3,707,535, 3,772,269; 3,839,318; 3,974,138; 4,223,129 and 4,528,106. Examples of useful alkylglycosides include, for example APG 325 CS Glycoside® which is described as being a 50% C₉-C₁₁ alkyl polyglycoside, also commonly referred to as D-glucopyranoside, (ex. Henkel KGaA) and Glucopon® 625 CS which is described as being a 50% C₁₀-C₁₆ alkyl polyglycoside, also commonly referred to as a D-glucopyranoside, (ex. Henkel).

The treatment compositions may include one or more amphoteric surfactants, specifically the following: derivatives of secondary and tertiary amines having aliphatic radicals that are straight chain or branched, and wherein one of the aliphatic substituents contains from about 8 to 18 carbon atoms and at least one of the aliphatic substituents contains an anionic water-solubilizing group, e.g., a carboxy, sulfonate, or a sulfate group. Non-limiting examples of compounds falling within this description include: sodium 3-(dodecylamino)propionate, and sodium 3-(dodecylamino)propane-1-sulfonate. Further exemplary useful amphoteric surfactants include sarcosinates and taurates, amide sulfosuccinates, and betaines including phosphobetaines. Exemplary betaines include dodecyl dimethyl betaine, cetyl dimethyl betaine, and dodecyl amidopropyldimethyl betaine.

The treatment composition may also comprise one or more cationic surfactant constituents, especially preferably one cationic surfactants which provide an appreciable germicidal benefit. Non-limiting examples of preferred cationic surfactant compositions which may be included in the treatment compositions are those which provide an appreciable germicidal benefit, and especially preferred are quaternary ammonium compounds and salts thereof, which may be characterized by the general structural formula: 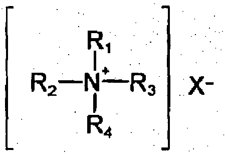 where at least one of R₁, R₂, R₃ and R₄ is a alkyl, aryl or alkylaryl substituent of from 6 to 26 carbon atoms, and the entire cation portion of the molecule has a molecular weight of at least 165. The alkyl substituents may be long-chain alkyl, long-chain alkoxyaryl, long-chain alkylaryl, halogen-substituted long-chain alkylaryl, long-chain alkylphenoxyalkyl, arylalkyl, etc. The remaining substituents on the nitrogen atoms other than the abovementioned alkyl substituents are hydrocarbons usually containing no more than 12 carbon atoms. The substituents R₁, R₂, R₃ and R₄ may be straight-chained or may be branched, but are preferably straight-chained, and may include one or more amide, ether or ester linkages. The counterion X may be any salt-forming anion which permits water solubility or water miscibility of the quaternary ammonium complex. Preferred quaternary ammonium compounds which act as germicides according to the foregoing formula are those in which R₂ and R₃ are the same or different C₈-C₁₂alkyl, or R₂ is C₁₂₋₁₆alkyl, C₈₋₁₈alkylethoxy, C₈₋₁₈alkylphenolethoxy and R₃ is benzyl, and X is a halide, for example chloride, bromide or iodide, or is a methosulfate anion. The alkyl groups recited in R₂ and R₃ may be straight-chained or branched, but are preferably substantially linear.

Particularly useful quaternary germicides include compositions which include a single quaternary compound, as well as mixtures of two or more different quaternary compounds. Such useful quaternary compounds are available under the BARDAC®, BARQUAT®, HYAMINE®, LONZABAC®, and ONYXIDE® trademarks, which are more fully described in, for example, McCutcheon's Functional Materials (Vol. 2), North American Edition, 1998, as well as the respective product literature from the suppliers identified below. When one or more cationic surfactants which provide an appreciable germicidal benefit are present, they may be present as a co-antimicrobial agent, with a further antimicrobial agent described hereinafter. When one or more cationic surfactants which provide an appreciable germicidal benefit are present, preferably anionic surfactants and further optionally, amphoteric surfactants are omitted from the treatment compositions of the invention. Other surfactants, although not specifically disclosed herein but known to the art may also be used within the treatment compositions of the present invention.

The treatment of the compositions may also include one or more fluorosurfactants. Preferred fluorocarbon surfactants include the anionic salts of perfluoroaliphaticoxybenzene sulfonic acids and the anionic salts of linear perfluoroalkyl-oxybenzoic acids. Examples of the former class of fluorocarbon surfactants can be represented by the following formula: 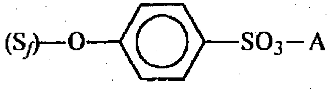 where R*_{f}* is a perfluoroaliphatic group of from about 5 to about 15 carbon atoms, preferably from about 8 to 12 carbon atoms in the aliphatic group which may be an alkyl group or alkenyl group, and A is a cation such as an alkali metal, ammonium or amine.

Examples of the latter class of fluorocarbon surfactants can be represented by the formula: 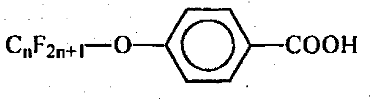 wherein n is a number of from about 2 to about 16 and m is a number from about 3 to about 34.

Other suitable fluorocarbon surfactants are:
(a) R*_{f}*CH₂CH₂SCH₂CO₂M wherein R*_{f}* is F(CF₂CF₂)ₙ and n is from about 3 to about 8 and M is alkali metal (e.g., sodium or potassium) or ammonium;
(b) CₙF₂ₙ₊₁SO₃M wherein CₙF₂ₙ₊₁ is a straight chain fluorocarbon radical, n is from about 8 to about 12 and M is alkali metal or ammonium;
(c) CₙF₂ₙ₊₁SO₃M wherein CₙF₂ₙ₊₁ is a straight chain fluorocarbon radical, n is from about 8 to about 12 and M is an alkali metal cation;
(d) R*_{f}*CH₂CH₂O(CH₂CH₂O)ₙH wherein R*_{f}* is a straight chain F(CF₂CF₂)ₙ radical and n is from about 3 to about 8;
(e) R*_{f}*(OCH₂CH₂)ₙOR*_{f}* wherein R*_{f}* is a branched chain radical of the formula C₈F₁₅+C₁₀F₁₉ or C₁₂F₂₃ and n is from about 10 to about 30; and
(f) R*_{f}*(OCH₂CH₂)ₘOR wherein R*_{f}* is a branched chain radical of the formula C₈F₁₅₊ C₁₀P₁₉ or C₁₂F₂₃, m is from about 2 to about 20 and R is C₁ to C₃ alkyl.

Fluorinated hydrocarbon surfactants arc available from numerous commercial sources as trademarked products. Examples are ZONYL fluorosurfactants from E.I. duPont de Nemours & Co., FLUORAD fluorosurfactants from 3M Company, e.g., FLUORAD FC-129 (R*_{f}*SO₂N(C₂H₅)CH₂CO₂⁻K⁺, where R*_{f}* is CₙF₂ₙ₊₁ and n is about 8), and MONOFLOR fluorocarbon surfactants from I.C.I. Americas, Inc. one or more such a fluorinated hydrocarbon surfactants maybe included in the treatment compositions and any desired for effective amount.

The treatment compositions may comprise further antimicrobial agents other than the one or more cationic surfactants described above. Such an antimicrobial agent is/are one or more compounds other than cationic surfactants which provide an appreciable germicidal benefit, viz., cationic germicide, described above. Such an antimicrobial agent desirably provides an effective antimicrobial benefit to a treated surface, other than a cationic germicide, preferably such that the treatment composition delivered by the device of the invention exhibits at least 3 log₁₀ kill efficacy, preferably at least 4 log₁₀ kill efficacy at 60 seconds contact time of at least two, preferably at least three and most preferably at least four of microorganisms selected from the group consisting of: *S*. *aureus*, *E. coli, P. aeruginosa* and *E.hirae*, desirably according accepted and standardized testing protocols for the evaluation of antimicrobial efficacy of a composition applied to a hard surface.

The antimicrobial agent may include one or more of: pyrithiones such as zinc pyrithione, halohydantoins such as dimethyldimethylol hydantoin, methylchloroisothiazolinone/methylisothiazolinone sodium sulfite, sodium bisulfite, imidazolidinyl urea, diazolidinyl urea, benzyl alcohol, 2-bromo-2-nitropropane-1,3-diol, formalin (formaldehyde), iodopropenyl butylcarbamate, chloroacetamide, methanamine, methyldibromonitrile glutaronitrile, glutaraldehyde, 5-bromo-5-nitro-1,3-dioxane, phenethyl alcohol, o-phenylphenol/sodium o-phenylphenol, sodium hydroxymethylglycinate, polymethoxy bicyclic oxazolidine, dimethoxane, thimersal dichlorobenzyl alcohol, captan, chlorphenenesin, dichlorophene, chlorbutanol, glyceryl laurate, halogenated diphenyl ethers such as 2,4,4-trichloro-2-hydroxy-diphenyl ether (Triclosan®) and 2,2-dihydroxy-5,5-dibromo-diphenyl ether, phenolic antimicrobial compounds such as mono- and poly-alkyl and aromatic halophenols, such as p-chlorophenol, methyl p-chlorophenol, 4-chloro-3,5-dimethyl phenol, 2,4-dichloro-3,5-dimethylphenol, 3,4,5,6-terabromo-2-methylphenol, 5-methyl-2-pentylphenol, 4-isopropyl-3-methylphenol, para-chloro-meta-xylenol, dichloro meta xylenol, chlorothymol, and 5-chloro-2-hydroxydiphenylmethane, resorcinol and its derivatives, bisphenolic compounds such as 2,2-methylene bis (4-chlorophenol) and bis (2-hydroxy-5-chlorobenzyl)sulphide, benzoic esters (parabens), halogenated carbanilides such as 3-trifluoromethyl-4,4'-dichlorocarbanilide (Triclocarban), 3-trifluoromethyl-4,4-dichlorocarbanilide and 3,3,4-trichlorocarbanilide.

The antimicrobial agent may include one or more of: biguanides such as polyhexamethylene biguanide, p-chlorophenyl biguanide; 4-chlorobenzhydryl biguanide, 1,6-bis-(4-chlorobenzylbiguanido)-hexane (Fluorhexidine®), halogenated hexidine including, but not limited to, chlorhexidine (1,1'-hexamethylene-bis-5-(4-chlorophenyl biguanide) (Chlorohexidine®), as well as salts of any of the foregoing, e.g. polyhexamethylene biguanide hydrochloride.

The treatment compositions of the invention may also comprise one or more organic or inorganic acids which may be used to adjust the pH of the treatment composition to a target range or level, and/or to impart an antimicrobial benefit. The acids may be one or more of a water soluble inorganic acids, mineral acids, or organic acids, with virtually all such known materials contemplated as being useful in the treatment compositions. By way of non-limiting example useful inorganic acids include mineral acids, hydrochloric acid, phosphoric acid, sulfuric acid, and the like.

In certain embodiments, the inventive compositions comprise one or more organic acids which also provide an antimicrobial benefit. Exemplary organic acids are those which generally include at least one carbon atom, and include at least one carboxyl group (--COOH) in its structure. Derivatives of said organic acids are also contemplated to be useful. Exemplary organic acid include linear aliphatic acids such as acetic acid; dicarboxylic acids, acidic amino acids, and hydroxy acids such as glycolic acid, lactic acid, hydroxyacrylic acid, alpha-hydroxybutyric acid, glyceric acid, malic acid, tartaric acid and citric acid, as well as acid salts of these organic acids. Of these, citric acid, sorbic acid, acetic acid, boric acid, formic acid, maleic acid, adipic acid, lactic acid, malic acid, malonic acid, glycolic acid, salicylic acid and/or derivatives thereof, e.g., salicylic acid derivatives such as esters of salicylic acid, such as ethylhexyl salicylate, dipropylene glycol salicylate, TEA salicylate, salicylic acid 2-ethylhexylester, salicylic acid 4-isopropyl benzylester, salicylic acid homomenthylester are preferred. Of course mixtures of one or more acids are contemplated as being useful.

The treatment composition may comprise one or more polyols as well, especially preferably where such one or more polyols are present within the treatment composition in amounts which are effective in imparting a sanitizing or disinfecting benefit to surfaces upon which the treatment compositions are applied. By way of non-limiting example, preferred are polyols containing from 2 to about 6 hydroxyl groups. Preferred polyols are water soluble. Specific, though non-limiting examples of polyols include: ethylene glycol, propylene glycol, glycerol, diethylene glycol, triethylene glycol, dipropylene glycol, tripropylene glycol, hexylene glycol, butylene glycol and when present, the polyols should be present in a sufficient concentration such the antimicrobial constituent of which they form at least a part, provides an effective sanitizing or disinfecting benefit to surfaces being treated with the treatment compositions.

The treatment composition may comprise a peroxygen compound which may be essentially any compound containing a dioxygen (O--O) bond. Dioxygen bonds, particularly bivalent O--O bonds, are readily cleavable, thereby allowing compounds containing them to act as powerful oxidizers. Non-limiting examples of classes of peroxygen compounds include peracids, peracid salts, and peroxides such as hydrogen peroxide. The peroxygen can be any aliphatic or aromatic peracid (or peroxyacid) that is functional for disinfectant purposes in accordance with embodiments of the present invention. While any functional peroxyacid can be used, peroxyacids containing from 1 to 7 carbons are the most practical for use. These peroxyacids can include, but not be limited to, peroxyformic acid, peroxyacetic acid, peroxyoxalic acid, peroxypropanoic acid, perlactic acid, peroxybutanoic acid, peroxypentanoic acid, peroxyhexanoic acid, peroxyadipic acid, peroxycitric, and/or peroxybenzoic acid. Exemplary peracid salts include permanganates, perborates, perchlorates, peracetates, percarbonates, persulphates, and the like. Exemplary peroxide compounds include hydrogen peroxide, metal peroxides and peroxyhydrates. The metal peroxides that can be used include, but are not limited to, sodium peroxide, magnesium peroxide, calcium peroxide, barium peroxide, and/or strontium peroxide. Other salts (for example sodium percarbonate) have hydrogen peroxide associated therewith are also considered to be a source of hydrogen peroxide, thereby producing hydrogen peroxide in situ.

The treatment compositions of the invention may also include an oxidizing agent which may be a halogen bleach. Preferably, the oxidizing agent is a halogen bleach source which may be selected from various hypohalite-producing species, for example, bleaches selected from the group consisting of the alkali metal and alkaline earth salts of hypohalite, haloamines, haloimines, haloimides and haloamides. All of these are believed to produce hypohalous bleaching species in situ. Preferably, the oxidizing agent is a hypohalite or a hypohalite generator capable of generating hypohalous bleaching species. Hereafter, the term "hypohalite" is used to describe both a hypohalite or a hypohalite generator, unless otherwise indicated. Preferably, the hypohalite oxidizing agent is a hypochlorite or a generator of hypochlorite in aqueous solution, although hypobromite or a hypobromite generator is also suitable. Representative hypochlorite generators include sodium, potassium, lithium, magnesium and calcium hypochlorite, chlorinated trisodium phosphate dodecahydrate, potassium and sodium dichloroisocyanurate and trichlorocyanuric acid. Organic bleach sources suitable for use include heterocyclic N-bromo and N-chloro imides such as trichlorocyanuric and tribromocyanuric acid, dibromocyanuric acid and dichlorocyanuric acid, and potassium and sodium salts thereof, N-brominated and N-chlorinated succinimide, malonimide, phthalimide and naphthalimide. Also suitable are hydantoins, such as dibromodimethyl-hydantoin and dichlorodimethyl-hydantoin, chlorodimethylhydantoin, N-chlorosulfamide (haloamide) and chloramine (haloamine). When present, advantageously the hypohalite oxidizing agent is an alkali metal hypochlorite, an alkaline earth salt of hypochlorite, or a mixture thereof.

The treatment compositions of the invention may also include a material which provides an air treatment technical benefit. A way of nonlimiting examples, such include fragrances, perfumes, compositions for the control or eradication of airborne insects, odor neutralizing agents, odor masking agents, as well as those which may impart holistic or aromatherapy benefits.

A fragrance may form part of the treatment composition, and which may be based on natural and synthetic fragrances and most commonly are mixtures or blends of a plurality of such fragrances, optionally in conjunction with a carrier such as an organic solvent or a mixture of organic solvents in which the fragrances are dissolved, suspended or dispersed. Typically, a fragrance is derived from one or more row raw materials which may be divided into three main groups: (1) the essential oils and products isolated from these oils; (2) products of animal origin; and (3) synthetic chemicals.
By way of non-limiting example, natural fragrances as well as certain essential oils include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, .alpha.-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable fragrance. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, labolanum oil and lavendin oil. When present such a fragrance constituent may be present in the treatment composition in any effective amount. Advantageously, the fragrance or perfume is present in amounts of from about 0.00001%wt. to about 50%wt. based on the total weight of the treatment composition of which they form a part, although, due to the mode of delivery of the mist generator means to which does not impart thermal degradation of such a constituent, its inclusion in even higher amounts to about 100%wt. of the treatment composition are also contemplated as being possible and indeed advantageous in certain embodiments of the invention.

The treatment composition of the invention may include one or more holistic constituents, particularly may include one or more essential oils which are selected to provide a so-called "aromatherapy benefit" to the user. Such essential oils are frequently extracted from naturally occurring botanical sources such as flowers, stems, leaves, roots and barks of aromatic plants. While essential oils may be used singly, it is also common to utilize blends of essential oils in order to provide a conjunctive aroma benefit, and possibly a therapeutic benefit as well. Similarly to fragrance compositions which may also include one or more essential oils, frequently, due to their potency, essential oils are often supplied dispersed in a liquid carrier such as in one or more organic solvents in which the essential oils are dissolved or dispersed. Preferred essential oils providing an aromatherapy benefit include one or more selected from chamomile oil, lavendin oil, lavender oil, grapefruit oil, lemon oil, line oil, mandarin orange oil, orange flower oil and orange oil. When present, these one or more essential oils providing an aromatherapy benefit are present in any effective amount, advantageously are present in amounts of from about 0.00001%wt. to about 50%wt. based on the total weight of the treatment composition of which they form a part, although, due to the mode of delivery of the mist generator means to which does not impart thermal degradation of such a holistic constituent or essential oils, their inclusion in even higher amounts to about 100%wt. of the treatment composition are also contemplated as being possible and indeed advantageous in certain embodiments of the invention. It is to be understood that these one or more essential oils providing an aromatherapy benefit may be used with our without the optional fragrancing constituent recited previously and alternately, may be used wholly or partially in place of said fragrancing constituent.

To maintain or establish a desired pH of a treatment composition, the use of one or more pH buffers is contemplated. The treatment compositions according to the invention optionally but desirably include an amount of a pH adjusting agent or pH buffer composition. Such compositions include many which are known to the art and which are conventionally used. By way of non-limiting example pH adjusting agents include phosphorus containing compounds, monovalent and polyvalent salts such as of silicates, carbonates, and borates, certain acids and bases, tartrates and certain acetates. Further exemplary pH adjusting agents include mineral acids, basic compositions, and organic acids, which are typically required in only minor amounts. By way of further non-limiting example pH buffering compositions include the alkali metal phosphates, polyphosphates, pyrophosphates, triphosphates, tetraphosphates, silicates, metasilicates, polysilicates, carbonates, hydroxides, and mixtures of the same. Certain salts, such as the alkaline earth phosphates, carbonates, hydroxides, can also function as buffers. It may also be suitable to use as buffers such materials as aluminosilicates (zeolites), borates, aluminates and certain organic materials such as gluconates, succinates, maleates, and their alkali metal salts. Desirably the compositions according to the invention include an effective amount of an organic acid and/or an inorganic salt form thereof which may be used to adjust and maintain the pH of the treatment compositions of the invention to the desired pH range. Particularly useful is citric acid and metal salts thereof such as sodium citrate which are widely available and which are effective in providing these pH adjustment and buffering effects.

The treatment compositions of the invention may also include one or more alkanolamines which in addition to providing an improved cleaning benefit may also be used to concurrently adjust the pH of the treatment composition. By way of nonlimiting examples such include monoalkanolamines, dialkanolamines, trialkanolamines, and alkylalkanolamines such as alkyl-dialkanolamines, and dialkyl-monoalkanolamines. The alkanol and alkyl groups are generally short to medium chain length, that is, from 1 to 7 carbons in length. For di- and trialkanolamines and dialkyl-monoalkanolamines, these groups can be combined on the same amine to produce for example, methylethylhydroxypropylhydroxylamine. One of skill can readily ascertain other members of this group.

The treatment compositions of the invention may also comprise one or more hydrotropes, preferably one or more anionic hydrotrope compounds. Exemplary hydtropes include, e.g., , benzene sulfonates, naphthalene sulfonates, C₁-C₁₁ alkyl benzene sulfonates, naphthalene sulfonates, C₅-C₁₁ alkyl sulfonates, C₆-C₁₁ alkyl sulfates, alkyl diphenyloxide disulfonates, and phosphate ester hydrotropes. The hydrotropic compounds of the invention are often provided in a salt form with a suitable counterion, such as one or more alkali, or alkali earth metals, such as sodium or potassium, especially sodium. However, other water soluble cations such as ammonium, mono-, di- and tri-lower alkyl, i.e., C₁₋₄ alkanol ammonium groups can be used in the place of the alkali metal cations. Exemplary alkyl benzene sulfonates include, for example, isopropylbenzene sulfonates, xylene sulfonates, toluene sulfonates, cumene sulfonates, as well as mixtures thereof. Exemplary C₅-C₁₁ alkyl sulfonates include hexyl sulfonates, octyl sulfonates, and hexyl/octyl sulfonates, and mixtures thereof. Particularly useful hydrotrope compounds include benzene sulfonates, o-toluene sulfonates, m-toluene sulfonates, and p-toluene sulfonates; 2,3-xylene sulfonates, 2,4-xylene sulfonates, and 4,6-xylene sulfonates; cumene sulfonates, wherein such exemplary hydrotropes are generally in a salt form thereof, including sodium and potassium salt forms.

According to a further aspect of the invention, there is provided a method for the treatment of lavatory surfaces and particularly preferably the surfaces of lavatory appliances, which method comprises the step of: providing a device which generates a mist of a treatment composition, which treatment composition contacts the hard surface and provides a technical benefit thereto. Typically, the treatment compositions delivered by the device according to this method comprise one or more solvents such as water and/or organic solvents, and one or more further constituents especially one or more surfactants or other materials which provide one or more desired technical benefits. Typically, the technical benefits provided are one or more of: a cleaning benefit, a disinfecting benefit, a sanitizing benefit, a bacteriostatic effect, an anti-viral benefit, a sporicidal benefit to reduce the presence of, incidence of or regrowth of molds, fungi, spores and the like, an anti-allergen benefit, an anti-acaricidal benefit, an anti-fungal benefit, an anti-resoiling benefit, a surface treatment benefit to improve the appearance thereof, e.g., surface shine and the like to the treated lavatory surfaces, particularly to resist subsequent staining of such treated surfaces.

In a still further aspect of the invention there is provided a method for the delivery of an air treatment composition to an airspace proximate to a lavatory appliance, which method comprises the step of providing a device which generates a mist of a treatment composition, which treatment composition contacts said airspace and provides a technical benefit thereto. Typically, the technical benefits provided are one or more of: fragrancing, perfuming, odor neutralizing, malodor treating or masking, air sanitization and the like.

In addition thereto, or alternately thereto treatment of an airspace can be achieved independently of the mist generator, which can be operated separately in order to provide a treatment composition, by the use of an air-treatment means which provides a volatile material to the ambient environment of the device. For example, a separate air-treatment means can be provided to as a module, or element werein such a module may contain a quantity of an air treatment composition from which it emanates to the ambient environment of the lavatory appliance. Such may include a porous solid material, e.g, a ceramic or polymeric material, a gel, a fibrous substrate such as a wick or pad and the like which can be operated to actively or passively permit for the volatilization of one or more chemical compounds in order to provide the air treatment composition to a lavatory, or to the environment proximate to a lavatory appliance treated by a device of the invention.

In a still further aspect of the invention there is provided a method for the delivery of a nebulized or atomized fluid treatment composition, viz., a "treatment mist" to a surface, or to an enclosed cavity, volume, or space associated with a lavatory appliance, viz., the interior of a toilet bowl, urinal or bidet.

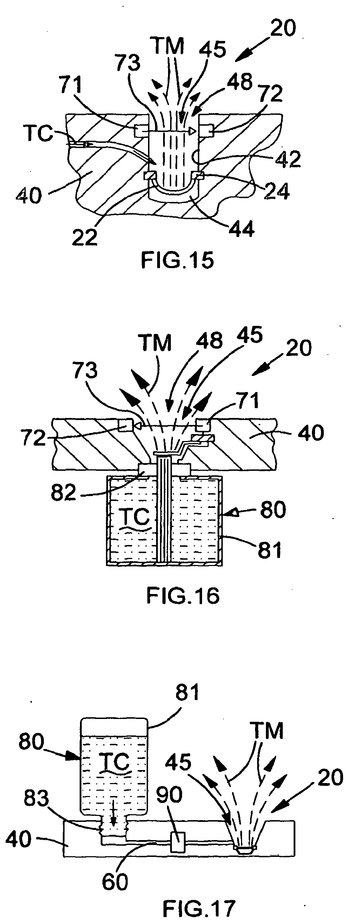

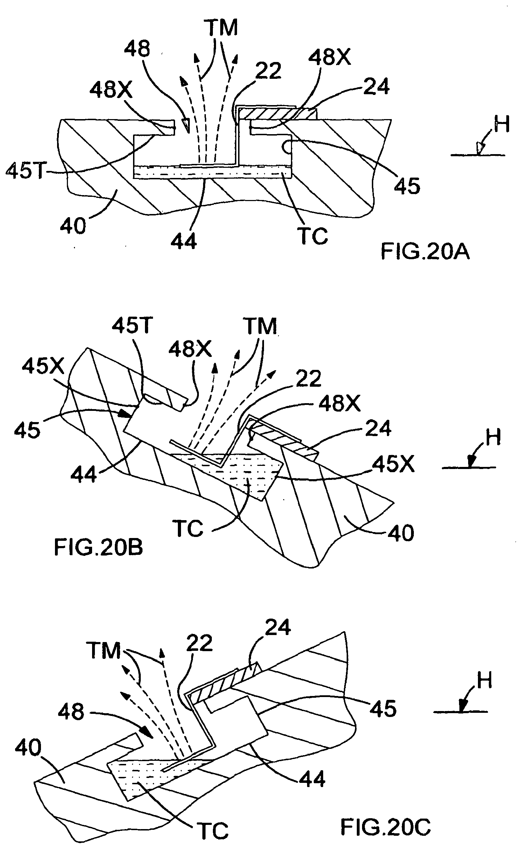

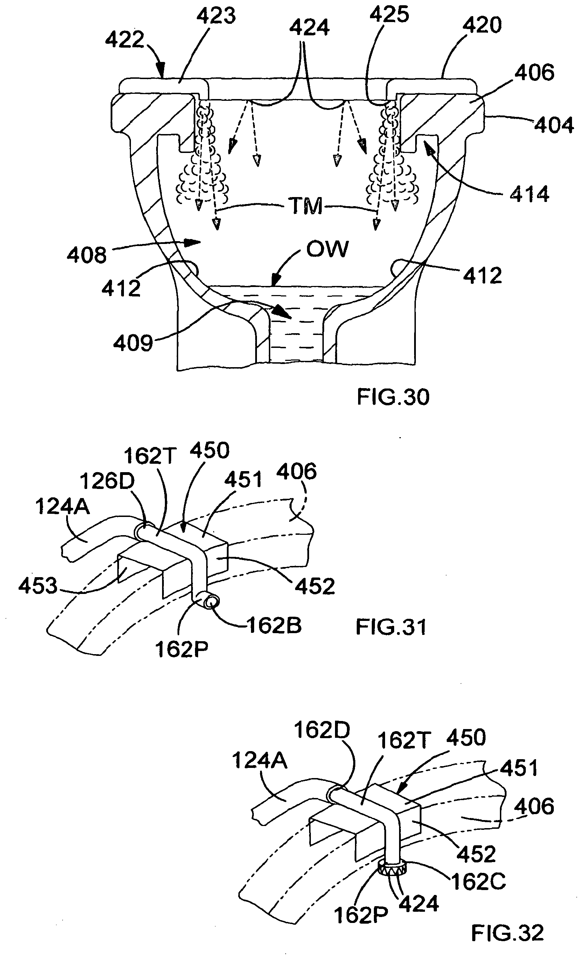

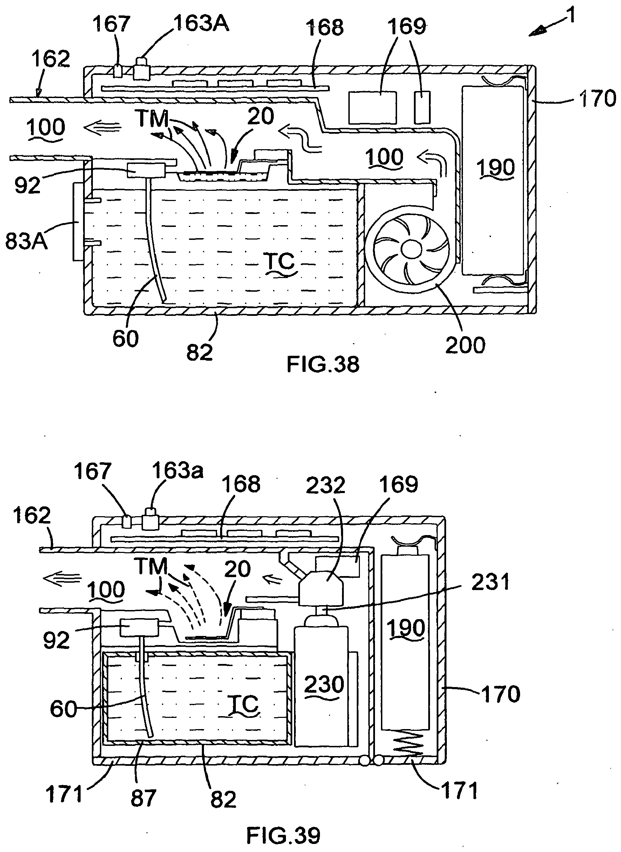

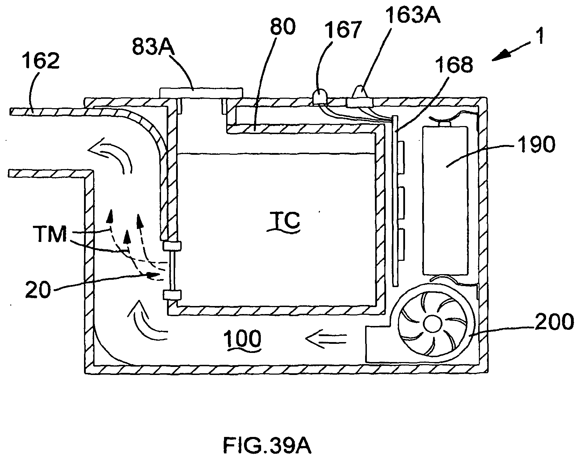

Reference is now made to the drawings, some of which illustrate various embodiments of the invention, including certain preferred embodiments of the invention. Other Figures, which show embodiments not covered by the claims, are included to provide a better understanding of the invention. In the accompanying figures, like elements are indicated using like numerals throughout the figures.

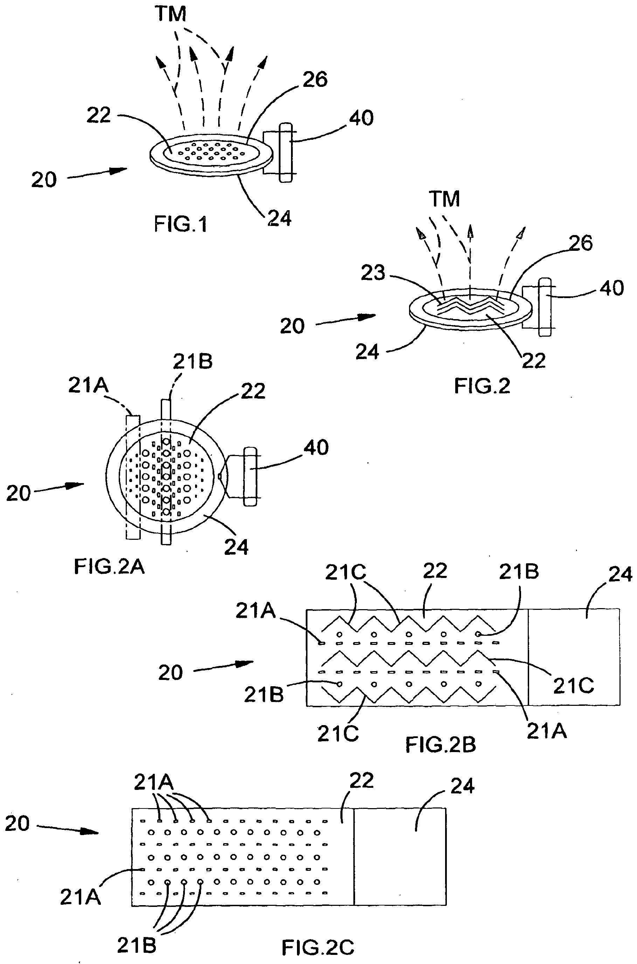

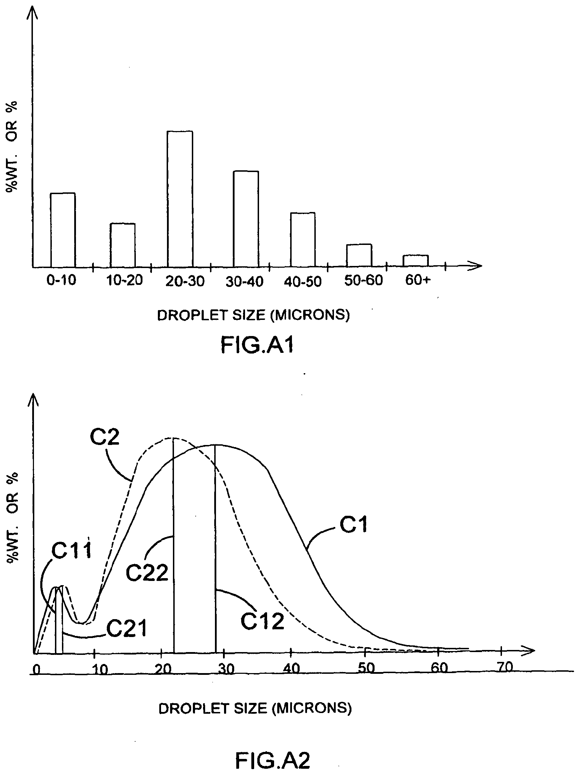

Figure 1 depicts an embodiment of a mist generator means 20 which comprises a vibrating plate 22, here formed of a micro-perforated metal screen or sheet. The vibrating plate 22 is generally circular, and includes a peripheral piezoelectric element 24. Although depicted in the embodiment that the piezoelectric element is at the peripheral edge 26 of the vibrating plate 22 and is affixed thereto, it is to be understood that the piezoelectric element 24 may be affixed to any part of the vibrating plate 22 and is not necessarily required to be at the periphery thereof. Further illustrated on the figure are a pair of electrical current carrying means 40, or, namely a pair of wires which supply an electrical current from the circuit control means (not shown) which acts to operate the mist generator means 20 by inducing the vibrations within that the vibrating plate 22 which acts to pump the treatment composition from the vibrating plate 22 as is indicated by reference arrows "TM" .
Figure 2 depicts an alternative embodiment of a mist generator means 20 which also comprises a vibrating plate 22, however in the present embodiment to series of segments 23 pass through the vibrating plate. Reference is made to US 7229028, Similarly, a piezoelectric element 24 is similarly illustrated at the peripheral age 26 of the vibrating plate 22 and is likewise affixed to thereto. Also illustrated is current carrying means 40, namely a pair of wires are also illustrated for providing means to transmit an electrical current to the piezoelectric element 24 from the circuit control means (not shown) to induce vibrations within the mist generator means 20 so to pump a treatment composition in the form of a mist TM in the direction of the reference arrows TM.
Figures 2A, 2B and 2C depict embodiments of a mist generator means 20 of different configurations which are adapted to provide a bi-modal distribution of liquid droplets or particles, viz., a treatment mist of the treatment composition. The embodiment presented on Fig. 2A is similar in most respects to the embodiments according to Figs. 1 and 2, but differ in that the vibrating plate 22 formed of a micro-perforated metal screen or sheet comprises a first series of microperforations 21A passing therethrough and a second series of microperforations 21B passing therethrough, which are of different configurations or sizes, e.g., cross section or diameters, the microperforations of each series being of different configurations or sizes, e.g., cross section or diameters than those of a different series. Treatment composition being nebulized by the mist generator means 20 is provided as a treatment mist having a bi-modal distribution of liquid droplets or liquid particles. The embodiment of Fig. 2B illustrates a further embodiment of a mist generator means 20 having a rectangular configuration, and includes a vibrating plate 22 formed of a micro-perforated metal screen or sheet comprises a first series of microperforations 21A passing therethrough, a second series of microperforations 21B passing therethrough, and a third series of microperforations 21C passing therethrough, the microperforations of each series being of different configurations or sizes, e.g., cross section or diameters than those of another series; treatment composition being nebulized by the mist generator means 20 is provided as a treatment mist having a three-modal distribution of liquid droplets or liquid particles. The embodiment of Fig. 2C illustrates a further embodiment of a mist generator means 20 having a rectangular configuration, and includes a vibrating plate 22 formed of a micro-perforated metal screen or sheet comprises a first series of microperforations 21A passing therethrough, and a second series of microperforations 21B passing therethrough; the microperforations of each series being of different configurations or sizes, e.g., cross section or diameters than those of another series; treatment composition being nebulized by the mist generator means 20 is provided as a treatment mist having a bi-modal distribution of liquid droplets or liquid particles.

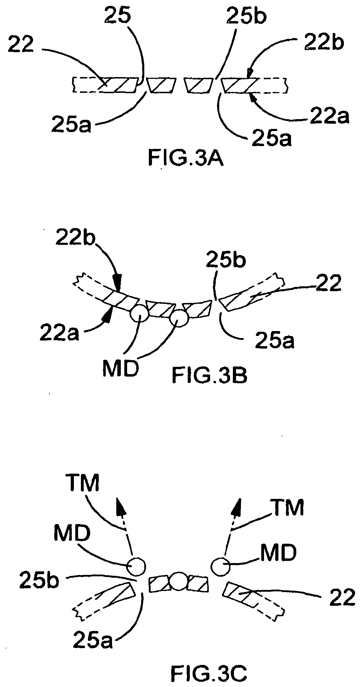

Figures 3A, 3B and 3C illustrate in a more detailed, cross-sectional view the operation of a portion of a vibrating plate 22 under normal operating conditions. Typically, when an appropriate electrical current is passed through the piezoelectric element 24, such induces the configuration, or the expansion and contraction of the piezoelectric element 24. The vibrating plate 22, at least a part of which is mechanically, chemically, or otherwise physically bonded to at least a part of the piezoelectric element 24 similarly vibrates but to due to the more flexible nature of the vibrating plate 22, an oscillatory pattern is introduced in to the vibrating plate 22. Where the vibrating plate 22 is generally circular in nature and is bound on its periphery to the piezoelectric element 24, as is disclosed in Figures 1 and 2, typically a rippling waveform, which extends from the periphery and towards the center of the vibrating plate 22 manifests itself. However when the vibrating plate 22 is generally rectangular, or is bonded on only one of its sides or one of its ends to piezoelectric element 24, a typically rippling waveform which extends from the point of connection between vibrating plate 22 and the piezoelectric 24 is manifested. The latter is due to the fact that wherein the parts of the vibrating plate 22 are not mechanically bound, such provides for more freedom of movement of the vibrating plate 22 at such points thereon. Nonetheless, in such a configuration, the waveform induces flexure of the vibrating plate 22 such that during the passage of a wave, or part of a waveform across any point of the vibrating plate 22, the region surrounding such a point will bend either upwardly, or downwardly with respect to the same point, as compared to the condition of the same point when the vibrating plate 22 is in a static state. Figure 3A illustrates a transverse view of a small section of a vibrating plate 22 in such a static state. As is visible thereon, the vibrating plate 22 includes a series of perforations or channels 25 passing therethrough, which optionally but preferably have a slightly wider diameter or width of passage entries 25a at the bottom face 22a of the vibrating plate 22, and slightly narrower diameter or width of passage exits 25b at the top face 22b of the vibrating plate 22. Such is believed to improve the pumping action of the treatment composition being transferred through the vibrating plate 22 when it operates as part of the mist generator means 20. Turning now to Fig. 3B, the same portion of the vibrating plate 22 is illustrated in the condition as being a "trough" of a portion of the waveform during the oscillation of the vibrating plate 22. Depicted are also pair of microdroplets "MD" of the treatment composition which are present at the passage entries 25a at the bottom face 22a of the vibrating plate 22. Such for example may be formed by the presence of a treatment composition beneath the vibrating plate 22, such as when supplied in a liquid form. Turning now to Fig. 3C, the same portion of the vibrating plate 22 is illustrated in the condition as it being at a "peak" of a portion of the waveform during the oscillation of the vibrating plate 22. As is visible thereon, the direction of flexure of the vibrating plate is now reversed with respect to that as illustrated on Fig. 3C, and as it is in an outwardly bowed direction perspective thereto, the passage exits 25b have a somewhat increased width or diameter as compared to one of the vibrating plate 22 was in the trough position, via., as per Fig. 3B or even when in a static position, as per Fig. 3A. Concurrently, the diameter or width of the passage entries 25a at the bottom face 22a of the vibrating plate 22 are reduced as compared to one of the vibrating plate 22 was in the trough position, via., as per Fig. 3B or even when in a static position, as per Fig. 3A, and such causes the microdroplets MD of the treatment composition to be expelled outwardly from the vibrating plate 22 in the direction of reference arrows TC. In such a manner, pumping of a liquid composition, here the treatment composition of the invention can be achieved across the thickness of the vibrating plate 22.
   It is however to be noted that while the provision of pumping across the thickness of the vibrating plate 22 provides an excellent means of atomizing the treatment composition and thereby providing a treatment composition in a form of a mist, it is foreseen that the treatment composition can alternately be supplied directly to the top face 22b of the vibrating plate 22, and due to the vibratory oscillation of the vibrating plates 22, microdroplets MD of the treatment composition are also formed without necessarily passing through the vibrating plate 22 as described immediately above.

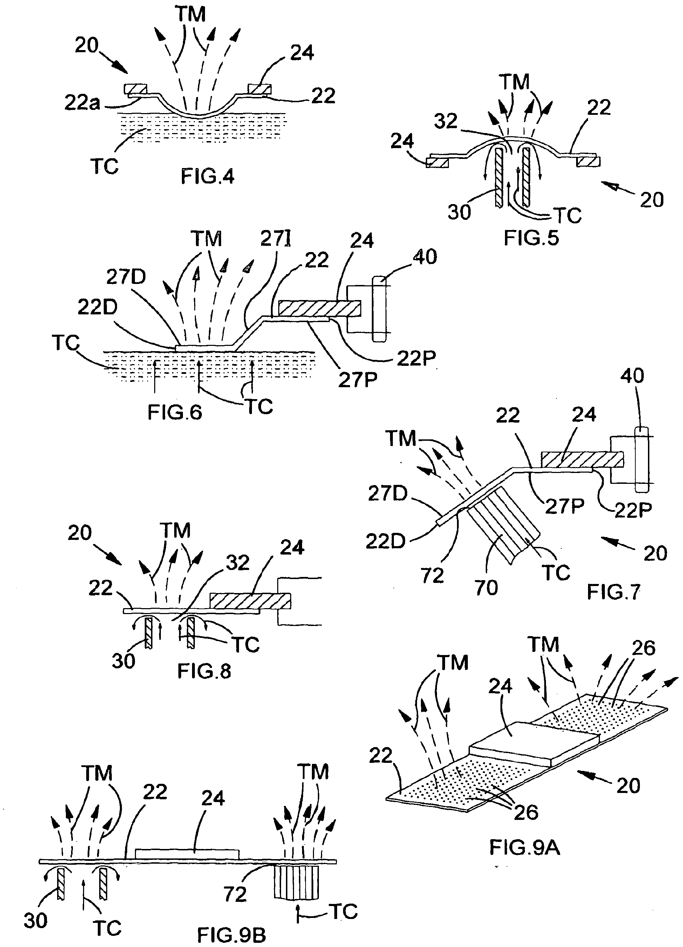

Figure 4 depicts a further embodiment of a vibrating plate 22, similar in several respects to the embodiments illustrated on Figs. 1 and 2. Thereupon is illustrated a mist generator means 20 which comprises a vibrating plate 22, here formed of a bowl shaped micro-perforated metal screen or sheet. The vibrating plate 22 is generally circular, and includes a peripheral piezoelectric element 24. A portion of the bottom face 22a is in contact with the surface of, or is partially immersed with the treatment composition TC, here in the form of a liquid. When operating, the mist generator means 20 pumps microdroplets of the treatment composition outwardly from the interior of the bowl shaped vibrating plate 22, upwardly an outwardly in the direction of reference arrows TM.
Figure 5 depicts a further embodiment of a mist generator means 20. In the depicted in embodiment, there is provided a vibrating plate 22, here formed of a bowl shaped micro-perforated metal screen or sheet which is generally circular and includes a peripheral piezoelectric element 24. A portion of the bottom face 22a is in contact with a surface of, or partially immersed with the treatment composition TC, here in the form of a column of flowing liquid supplied by a fluid conduit 30, here a circular tube 30. The treatment composition TC flows out from the open end 32 of the tube 30 and maintains a meniscus or layer of the treatment composition at this open end 32. When operating, the mist generator means 20 pumps microdroplets MD of the treatment composition outwardly from the interior of the bowl shaped vibrating plate 22, upwardly and outwardly in the direction of reference arrows TM, as during part of its oscillation, the vibrating plate 22 comes into contact with the treatment composition TC and pumps it through and outwardly from the vibrating plate 22 in the manner described previously. The quantity of the treatment composition which exits the tube 30 can be recirculated to resupply the vibrating plate 22 or alternately, can be collected or drained off and discarded.
Fig. 6 depicts an alternative embodiment of a mist generator means 20. The mist generator means 20 comprises a piezoelectric element 24 and a vibrating plate 22, here formed of a micro-perforated metal screen or sheet which is generally rectangular in configuration. In the present embodiment, only one end of the vibrating plate 22 on bonded to piezoelectric element 24, and during operation of the mist generator means 20 a typically rippling waveform which extends from the a proximal end 22P of the vibrating plate 22 along its length to its distal end 22D, is manifested. The latter is due to the fact that as parts of the vibrating plate 22 are not mechanically bound, particularly in the distal end 22D such provides for more freedom of movement of the vibrating plate 22. In the illustrated embodiment, while the vibrating plate 22 is generally rectangular it also is bent thus to define 3 interconnected parts, a proximal end part 27D, an intermediate part 271, and a distal end part 27D. In the depicted embodiment, the proximal end part 27P and distal end part 27D all are generally parallel but spaced apart from one another via the intermediate part 27I which is angled to both the proximal end part 27D and distal end part 27D. Here, the angles are approximately equal and approximately between 30 and 45 degrees of arc. Greater, and lesser angles are contemplated than the angles shown in the figure. Further illustrated on the figure are a pair of electrical current carrying means 40, or, namely a pair of wires which supply an electrical current from controller means (not shown) which acts to operate the mist generator means 20 by inducing the vibrations within that the vibrating plate 22 which acts to pump the treatment composition outwardly from vibrating plate 22 as is indicated by reference arrows "TM". As illustrated in this figure, the distal end part 27D of the vibrating plate 22 is in contact with, or immersed in a quantity of the treatment composition TC, here present in the form of a liquid. Although not visible in the drawings, the distal end part 27D includes passages or microperforations as discussed with reference to Figs. 1, 2, 2A, 2B or 2C. During operation of the mist generator means 20, oscillation of the vibrating plate 22 pumps microdroplets of the treatment composition outwardly from the vibrating plate 22, in the direction of reference arrows TM.
Figure 7 illustrates a further embodiment of a mist generator means 20 similar in some respects to the embodiment depicted on Fig. 6. In the present figure, a portion of a rectangular vibrating plate 22 is affixed, attached or bonded to a piezoelectric element 24, and the rectangular vibrating plate 22 extends outwardly therefrom. The vibrating plate 22 has a proximal end part 27P which extends the an intermediate angle to a distal end part 27D which comprises passages or microperforations as discussed with reference to Figs. 1 and 2. Thus the portion of the vibrating plate 22 comprising passages or microperforations is inclined. The treatment composition in the form of a liquid is supplied by a capillary means 70 here depicted as a porous fibrous element which transfers the treatment composition towards the terminal end 72 of the capillary means 70 from a reservoir containing the treatment composition (not shown). Alternatively, the porous fibrous element can be substituted by a ceramic element, or may be a plurality of thin diameter tubes such as a plurality of a thin diameter tubes which may be bundled to form a suitable capillary means 40. During operation, the capillary means 70 transmits the treatment composition word forms a film layer or meniscus at the terminal end 42 where it is retained. During the vibratory movement of the vibrating plate 22, the portion of the vibrating plate 22 comprising the passages or microperforations entrains, and thereafter pumps the treatment composition upward and outward from the vibrating plates 22 in the direction of reference arrows TM.
Figure 8 depicts an embodiment of a part of mist generator means 20 wherein a treatment composition TM is supplied to the vibrating plate 22 as a column of flowing liquid supplied by a fluid conduit 30, here a circular tube, wherein a sufficient amount of the treatment composition is present as a meniscus at the open end of the tube or may overflow. During part of its oscillation, the vibrating plate 22 comes into contact with the treatment composition TC and pumps it through an outwardly from the vibrating plate 22 and in the direction of reference arrows TM
Figure 9A depicts an embodiment of a mist generator means 20 adapted for use with dual sources of the treatment composition. As is visible thereon, a vibrating plate 22 of a generally rectangular configuration comprises a piezoelectric element 24 in its midsection. The vibrating plate 22 has two distal end parts 27D each of which comprises passages or microperforations as discussed with reference to Figs. 1 and 2, 2A, 2B and 2C.
   The operation of such a mist generator means 20 is more clearly disclosed on the side view presented in Fig. 9B which illustrates the mist generator means 20, and two sources of a treatment composition TC which can be the same or different. A first source of a treatment composition TC is a column of flowing liquid supplied by a fluid conduit 30, here a circular tube, wherein the treatment composition TC is delivered in the manner described in Fig. 8, such that during part of its oscillation, the vibrating plate 22 comes into contact with the treatment composition TC and pumps it through an outwardly from the vibrating plate 22 and in the direction of reference arrows TM. A second source of a treatment composition is wherein the treatment composition in the form of a liquid is supplied by a capillary means 70 which transfers the treatment composition towards the terminal end 72 of the capillary means 70 from a reservoir containing the treatment composition as discussed with reference to Fig. 9A. During the vibratory movement of the vibrating plate 22, the portion of the vibrating plate 22 comprising the passages or microperforations in trains, and thereafter pumps the treatment composition upward and outward from the vibrating plates 22 in the direction of reference arrows TM. In such an embodiment, a single piezoelectric element 24 and be used to induce vibration into one or more vibrating plates 22 each having one or more regions which comprise passages or microperforations as discussed with reference to Figs. 1 and 2, or a single vibrating plate having one or more regions which comprise comprises passages or microperforations as discussed with reference to Figs. 1 and 2, and consequently can be used to deliver one or more treatment compositions TC, which may be the same or different. For example, one treatment composition may be primarily provided as providing a treatment benefit to a surface, while the other treatment composition may be primarily provided to provide a treatment benefit to an airspace. Also coming into consideration is the provision of treatment compositions from devices according to the invention wherein a first treatment composition TC and a second different treatment composition TC are separately stored in separate reservoirs, but are simultaneously delivered when then coming into contact undergo a chemical reaction when in the form of a mist in order to form a treatment composition providing a technical benefit. Additionally it is contemplated that the vibrating plates 22 may have two or more differently sized or configures series of microperforations of each series being of different configurations or sizes, e.g., cross section or diameters than those of another series, as discussed with reference to Figs. 2A, 2B and 2C. The treatment composition or two different treatment compositions being nebulized by the mist generator means 20 according to Figs. 9A and 9B may be provided as a treatment mist having at least a bi-modal distribution of liquid droplets or liquid particles.

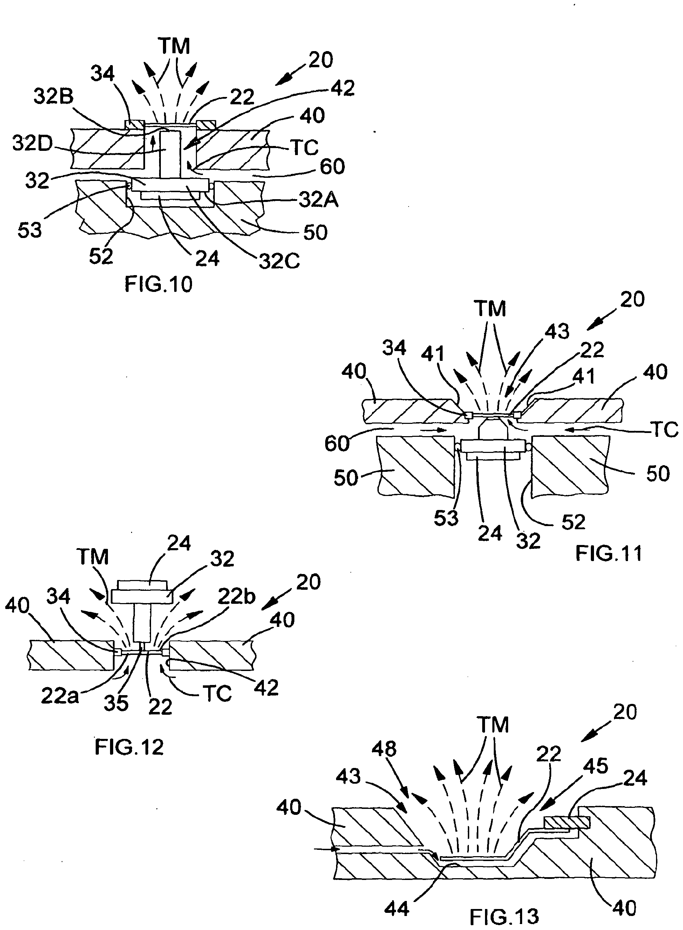

Figure 10 depicts a further embodiment of a mist generator means 20. Therein, a piezoelectric element 24 is mounted on a bottom face 32A of a transmission element 32 opposite from a top face 32B which is slightly spaced apart but in parallel to a vibrating plate 22. The vibrating plate 22 is mounted via a surrounding mounting frame 34 to a first body element 40 of a portion of the device. The surrounding mounting frame 34 is rigid and does not, per se, introduce any vibratory motion to the vibrating plate 22. This body element 40 comprises a circular bore 42 through which the upper part 32D of the transmission element 32 extends. In this depicted embodiment, the transmission element 32 is a generally circular and symmetrical about a vertical central axis extending through the center of the upper part 32D as well as lower part 32C to form a "stepped cylinder" as depicted. The lower part 32C is partially mounted within a bore 52 of a second body part 50 by a suitable mounting means here a peripheral O-ring 53 which is elastomeric thereby providing a liquid tight seal and yet at the same time permitting for movement of the transmission element 32 along its center axis and in the direction of the vibrating plate 22. A supply of the treatment composition TC is supplied to the circular bore 42 and the vibrating plate 22 via a fluid conduit 60 positioned between the first body element 40 and the second body element 50. Thus, the treatment composition TC may be supplied to the region between the top face 32B and the vibrating plate 22. When the piezoelectric element 24 is actuated, a vibratory motion is induced within the transmission element 32 which then oscillates alternately toward and away from the vibrating plate 22. Such motion causes pumping of the treatment composition TC through passages or microperforations present in the vibrating plate 22 such that a mist of the treatment composition TC is formed and expelled outwardly from the vibrating plate 22 in the direction of reference arrows TM. Such motion of the transmission element may also induce vibration in the vibrating plate as well, also causing pumping of the treatment composition TC therethrough and formation of a mist TM of the treatment composition TC. In the foregoing embodiment is to be understood that the reference to the first body element 40 and the second body element 50 need not be necessarily limited to discrete and separate portions of the device but can be a composite or a unitary element having in the appropriate configurations as described with reference to the figure.
Figure 11 illustrates a further embodiment of the mist generator means 20, similar in several respects to the embodiment according to prior Fig. 10. Therein, a first body part 40 includes a downwardly sloping circular sidewall 41 at or near the bottom of which is transversely mounted a vibrating plate 22 in a frame 34. The region within it the circular sidewall 41 and the vibrating plates 22 defines a weir 43 within which the treatment composition TC may be supplied or which may be collected. Beneath the vibrating plate 22 is a transmission element 32 in the form of a stepped cylinder, on the underside of which is mounted a piezoelectric element 24. Similarly to Fig. 10, the transmission element 32 is mounted within a circular bore 52 in a second body part 50 via a suitable mounting means, here an elastomeric seal 53 which is flexible but provides a liquid tight seal between the lower part 32C of the transmission element 32 and the bore 52. Treatment composition TC is supplied between the transmission element 32 and the vibrating plate 22 via a fluid conduit 60 positioned between the first body element 40 and the second body element 50. When the piezoelectric element 24 is actuated, a vibratory motion is induced within the transmission element 32 which then oscillates alternately toward and away from the vibrating plate 22, causing pumping of the treatment composition TC therethrough and thereby form a mist TM of the treatment composition.
Figure 12 illustrates a further embodiment of the mist generator means 20, similar in several respects to the embodiment according to prior Figs. 10 and 11. In the embodiment depicted, and first body part 40 includes a bore 42 passing the therethrough. A vibrating plate 22 is mounted via a peripheral mounting frame 34 transversely across a portion of the bore 42. Treatment composition TC is supplied to the underside of the vibrating plate 22 via a fluid conduit 60 where it contacts the bottom face 22a of the vibrating plate 22. On the opposite side of the vibrating plate 22 is mounted a transmission element 32. The transmission element 32 is in the form of a stepped cylinder, having a piezoelectric element 24 mounted at one end thereof, and at the opposite end thereof a pin 35 which is in physical contact with the top face 22b of the vibrating plate 22. While not shown, the transmission element 32 he suitably mounted by appropriate mounting means such that its pin 35 can oscillate into, and away from the vibrating plate 32 when the piezoelectric element 24 is actuated. Due to this physical contact between the pin 35 and the vibrating plate 22, a vibratory or oscillatory motion is induced within the vibrating plate 22, causing pumping of the treatment composition TC therethrough and thereby a mist TM of the treatment composition is formed..
Figure 13 depicts an alternative form of a mist generator means 20 useful in devices of the invention. Reference is made to US 20070169775, and US 20090121043. A first body part 40 includes an atomizing chamber 45, herein defined by a weir 43 and a base 44 within which is present a piezoelectric element 24 and a vibrating plate 22, here formed of a micro-perforated metal screen or sheet which is generally rectangular in configuration, which elements are described with reference to Fig. 6. A supply of the treatment composition TC enters the atomizing chamber 45 via fluid conduit within the first body part 40, and went the piezoelectric element 24 is actuated, the vibrating plate 22 vibrates or oscillates, thereby forming a mist TM of the treatment composition which is expelled outwardly from the atomizing chamber 45.

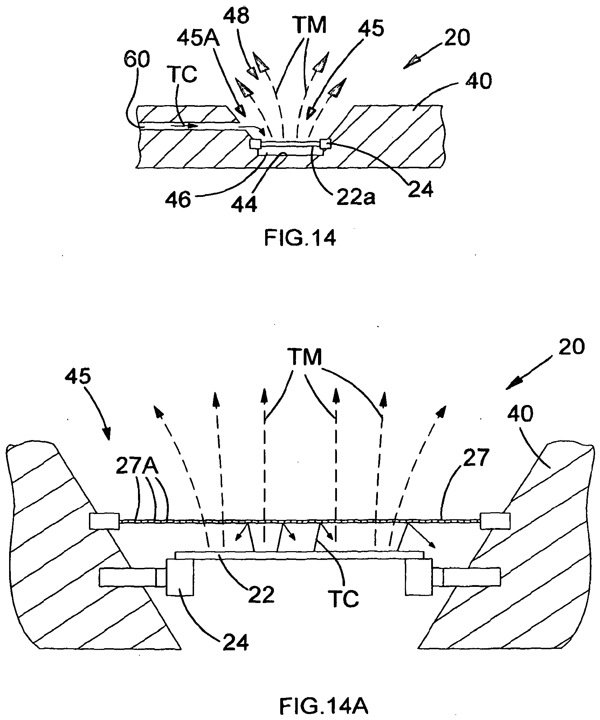

Figure 14 depicts a further alternative form of the mist generator means 20 useful in devices of the invention. A vibrating plate 22 bonded, mounted, or otherwise affixed to a peripheral piezoelectric element 24 generally is depicted in either of Figs. 1 or 2 is positioned slightly above the base 44 of a weir 43 present within a first body part 40 of the device. A fluid conduit 60 supplies a quantity of the treatment composition TC to the top face 22b of the vibrating plate 22. A small gap may exist between the bottom face 22a of the vibrating plate 22 and the base 44 thereby defining a base cavity 46. When the piezoelectric element 24 is actuated, the vibratory motion within the vibrating plate 22 causes the formation of a mist TM of atomized particles of the treatment composition TC within the atomizing chamber 45 which are expelled therefrom. Thus, the figure illustrates that the treatment composition TC need not necessarily be pumped through the vibrating plate in order to atomize the treatment composition TC. Advantageously, any liquid or fluid treatment composition TC which may collect within this base cavity 46 was ultimately atomized by the vibratory motion within the vibrating plate 22 which also exits the atomizing chamber 45.
Figure 14A illustrates a further embodiment of a mist generator means 20 useful in devices of the invention. A vibrating plate 22 which however only optionally but preferably includes microperforations passing therethrough as described with reference to Figs. 1, 2, 2A, 2B and 2C, is bonded, mounted, or otherwise affixed to a peripheral piezoelectric element 24 generally is depicted in either of Figs. 1 or 2 is positioned within an atomizing chamber 45. Parallel and spaced apart from the vibrating plate 22 is a perforated screen element 27 having a plurality of perforations 27A passing therethrough. In operation, the vibrating plate 22 operates to nebulizer the treatment composition into discrete droplets or particles which are directed towards the perforated screen element 27, however only those discrete droplets or particles not in excess of a specific droplet size or particle mass are expelled as a treatment mist TM, while the those discrete droplets or particles TC in excess of a specific droplet size or particle mass are returned to the vibrating plate 22. In this manner a controlled maximum particle size for the discrete droplets or particles of the treatment mist may be established.
   In the embodiments disclosed in Figures 13 and 14 and 14A, a bore, cavity or other configuration other than a weir with at least one sloping sidewall may be used as part of the atomizing chamber 45 as disclosed in several of the following figures.
With reference now to Fig. 15, therein is depicted a further embodiment of an atomizing chamber 45 present within the first body part 40, here a generally circular bore 42 having a base 44 opposite from an open and 48. Above the slightly concave shaped base 44 and mounted transversely across a portion of the bore 42 is a vibrating plate 22 and a piezoelectric element 24 as depicted on Fig. 4. A supply of the treatment composition TC enters the atomizing chamber 45 via fluid conduit and above the vibrating plate 22 such that it contacts the top face 22b. When the piezoelectric element is actuated, vibrations are induced within the vibrating plate 22 which causes the formation of a mist TM of atomized particles of the treatment composition TC within the atomizing chamber 45 which are expelled via the open end 48. Any liquid or fluid treatment composition TC which may collect between the vibrating plate 22 and the slightly concave shaped base 44 is also atomized by the vibratory motion within the vibrating plate 22 and also exits the atomizing chamber 45. Figure 15 also illustrates a sensor means, here a mist sensor means. In the instant embodiment the mist sensor means, includes a transmitter unit 71 and a receiver unit 72 mounted transversely from each other across the bore 45A and preferably near the open end 48 thereof. The transmitter unit generates a signals, e.g. such as optical, acoustic, or other signal capable of being received by the receiver unit, and any variations in the quality of the signal being transmitted due to the quantity or quality of the presence of the atomized particles, viz, mist, of the treatment composition passing through the gap between the transmitter unit 71 and receiver unit 72, as represented by arrow 73 is detected by the receiver unit. An appropriate signal can be transmitted to the controller means (not shown) which may initiate a responsive action by the controller means and one or more further parts of the device. For example, wherein the mist sensor means determines that an insufficient quantity of the atomized particles of the treatment composition are being produced, a signal representative of this state may be transmitted to the controller means which for example may increase the power or alternately increase the frequency signal being transmitted to the piezoelectric elements 24 to thereby increase the rate of its oscillation or vibration, and/or alternately the mass flow rate of the treatment composition TC, such as may be supplied via a pump, may be increased. Alternately, the mist sensor means may also determine if the atomizing chamber 45 is flooded with the fluid form of the treatment composition and upon sending a signal to the control unit representative thereof, the control unit may cause an appropriate response, e.g., shutting down of the device or interrupting the operation of the mist generating means 20. Still alternately, the mist sensor means may also determine the absence or presence of the mist of the treatment composition within the atomizing chamber 45, and if the latter is sensed then a representative signal may be sent to the control unit may cause an appropriate response, e.g., shutting down of the device or interrupting operation of the mist generating means 20.
Figure 16 illustrates a further embodiment of a portion of the device. A reservoir 80, here in the form of a hollow container 81 containing a quantity of the treatment composition in a fluid form, preferably in a liquid form, is removably affixed to a first body part 40 of the device. The reservoir 80 includes a cap 82 having passing therethrough a capillary means 70 here, a porous fibrous element which transfers the treatment composition towards the terminal end 72 of the capillary means 40. The body part 40 includes an atomizing chamber 45 similar in most respects to the embodiment depicted on Fig. 13, accepted that the base of the atomizing chamber is replaced by a portion of the 82 which forms a liquid tight seal with the first body part 40. Such also permits for the alignment of the terminal end 72 of the capillary means 70 such that due to capillary forces within the capillary means 40, a quantity of the treatment composition is continually presented to the terminal end 72 from which it may be atomized by the mist generator 20. The atomized particles of the treatment composition form a mist TM which exits via the open end 48 of the atomizing chamber 45. Also depicted is a mist sensor means comprising a transmitter unit 71 and a receiver unit 72 mounted transversely from each other across the bore 45 and preferably near the open end 48 thereof.
Figure 17 depicts an embodiment wherein the effect of gravity is used to deliver the treatment composition TC to the mist generator 20. A hollow container 81, viz., a bottle, having an open neck end 83 is inverted and mounted within the first body part 40 such that the treatment composition flows out from the inverted container 81 under the force of gravity. A fluid conduit 60 connects the open neck end 83 with an atomizing chamber 45 containing a mist generator 45 substantially as described with reference to Fig. 14. Intermediate and in line with the fluid conduit 60 is a fluid control means 90, which may be any device which may impart control over the quantity or quality of the fluid treatment composition passing therethrough. In the simplest embodiment, such can be any valve which can be either manually, what is desirably controlled by the controller means (not shown). Appropriate control of the fluid control means 90 may be used to ensure that an optimal supply of the fluid treatment composition TC is transmitted to the atomizing chamber 45 to ensure desired operation of the mist generator 20.
   Although not illustrated in the depictions, it is to be understood nonetheless that suitable electrical or signal unit conducting means, i.e. wires, may be used to connect the various elements of the mist sensor means, the fluid control means, the controller means, as well as any other device, elements or parts of the device as may be required, although such is not necessarily illustrated in the figures presented herein.
   Figures A1 and A2 illustrate by means of graphical representations preferred treatment mist particle size or particle mass bi-modal distributions. Figure A1 represents the mass distribution or % distribution of the size (in microns) of the discrete liquid droplets being dispensed by a mist generator, during normal steady state operation over a convenient time interval, e.g., 1 or more seconds, or one or more minutes.. As is seen thereon, a greater amount of particles in the range of 0 - 10 microns are dispensed than the amount of particles in the range of 10 - 20 microns, whereas the amount of particles in the successive ranges of 20-30 microns is greater than those dispensed in the prior two ranges. As particle sizes increase to higher ranges, viz., 30 - 40 microns, and 40 - 50 microns, their amounts decrease successively. As can also be seen from Fig. A1, the total mass of the dispensed particles in the range of 0 - 10, is substantially lesser than the total mass of the dispensed particles in the ranges of 20 microns and greater. Figure A2 illustrates two further alternative bi-modal distributions according to preferred embodiments of the invention, here represented as a first bi-modal distribution represented by "C1" (in solid line) and a second bi-modal distribution represented by "C2" (in dotted line). The curves represent the distribution, by %wt. or mass or percentage of respective discrete liquid droplets or particles of the treatment composition present in a treatment mist formed therefrom, as indicated on the y-axis, for droplets within a particular micron size range, as indicated on the x-axis. With reference to line C1, it is seen that the first median or first averaged liquid particle size corresponds to line segment C11, which is approximately at 4 microns with the particle size distribution within the first part of the bi-modal distribution being beneath the curved line C1 to the left and right of the line segment C11, and the second median or second averaged liquid particle size corresponds to line segment C12, which is at approximately 29 microns, with the particle size distribution within the second part of the bi-modal distribution being to the left and right of the line segment and beneath curved line C1. The further bi-modal distribution represented by C2 is similar in many respects but, first median or first averaged liquid particle size corresponds to line segment C21, which is approximately at 5 microns with the particle size distribution within the first part of the bi-modal distribution being beneath the curved line C2 to the left and right of the line segment C21, and the second median or second averaged liquid particle size corresponds to line segment C22, which is at approximately 22 microns, with the particle size distribution within the second part of the bi-modal distribution being to the left and right of the line segment and beneath curved line C2.

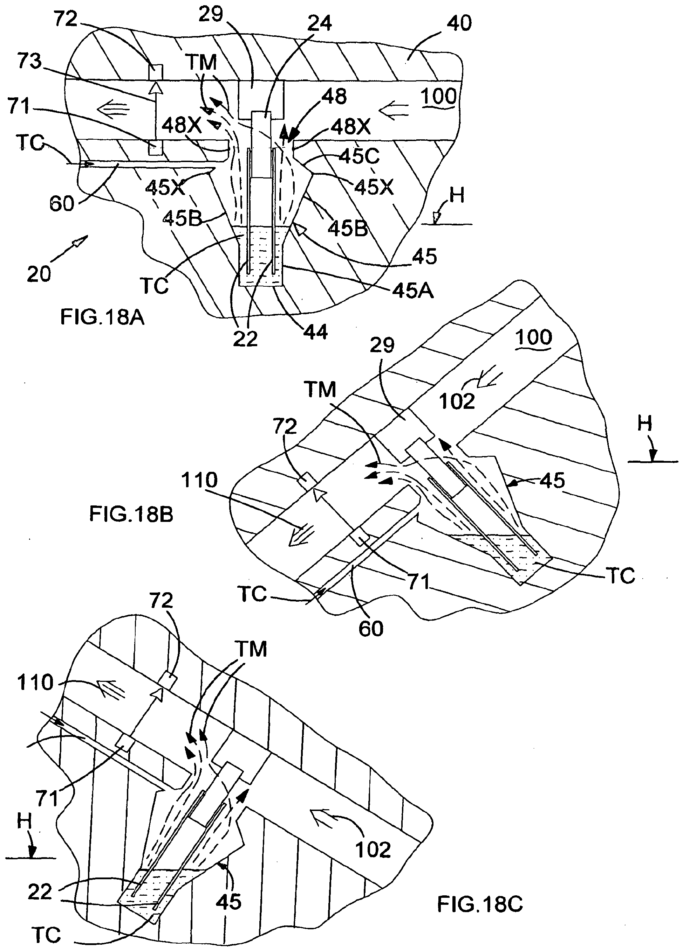

Figures 18A, 18B and 18C illustrate a portion of a device similar to the device of the invention and an embodiment of an atomizing chamber 45 which is resistant to spillage of the fluid treatment composition contained therein when tipped away from the horizontal, as represented by the line segment "H" in the figures. In Fig. 18A, the atomizing chamber 45 is oriented such that the pair of vibrating plates 22 affixed at one end thereof to a piezoelectric device 24 extend vertically, downwardly into a quantity of the treatment composition TC adjacent to the base 44 of the atomizing chamber 45. In this embodiment, the atomizing chamber 45 is concentric about a center axis running through the center of the base 44 upwardly and through the opening 48 at the opposite end of the atomizing chamber 45, although this is not a necessary requirement. The atomizing chamber 45 pass, directed upwardly from its base 44, a generally circular base portion 45A, which extends into an intermediate, outwardly extending frustroconical portion 45B, which extends into a next, reverse frustroconical portion 45C which extends inwardly and merges into the opening 48. As is seen thereon, these sections define an interior volume of the atomizing chamber 45 which is adapted to contain and at least the part thereof a quantity of the treatment composition TC, and at least a part of the mist generator 20. As is also seen, the interior mime of the atomizing chamber 45 also has a maximum, transverse cross-section or maximum transverse dimension which in the depict embodiment, can be defined as extending between opposing points 45X. This maximum transverse dimension is most preferably greater than the maximum transverse dimension of the opening 48. Advantageously, the height or distance between the base 44 is at least 1.1 times, preferably at least 1.2, and in order of increasing preference is at least: 1.3, 1.4. 1.5, 1.7, 2, 2.2, 2.5 times, or even greater than the maximum transverse dimension of the opening 48, which in the depict embodiment can be the distance between opposing points 48X. in such a manner, a "well shaped" and atomizing chamber 45 can be produced, and which has dimension such that when the device and/or the atomizing chamber 45 is tilted or reoriented from the horizontal, "H", such as in the orientations depicted on Fig. 18A and 18B, the volume of the treatment composition TC does not spill outwardly, via the opening 48 and out from the device but rather is retained within the atomizing chamber 45. Furthermore, in either such orientation wherein the atomizing chamber 45 is angled with respect to the horizontal, as opposed to the depiction of Fig. 18 wherein the atomizing chamber is perpendicular to the horizontal, at least a part of at least one of the vibrating plates 22 remains in contact with the treatment composition TC such that when the piezoelectric element 24 is energized, the vibrating plate or plates 22 atomized at the treatment composition TC which forms a treatment mist TM which exits via the opening 48 and enters the airflow conduit 100. As the mount 29 of the piezoelectric element 24, as well as the piezoelectric element 24 are configured to allow for the bypass of a stream of gas, preferably air, through the airflow conduit 100 and across the opening 48, the flowing gas represented by arrow 102, entrains the treatment mist 110 which flows towards an outlet (not shown) of the device
   It is to be noted that in the foregoing embodiments, while the fluid conduit 60 has been illustrated is being an integral portion of either a first part 40 or second part 50 other device, e.g, as a bore or channel, such as to be understood as being merely by way of illustration as any fluid directing means, including a separate channel, conduit, tubing, or pipe element, capable of transmitting the treatment composition in fluid form so to come in contact with the mist generator 20 is clearly contemplated and may be used in any environment of the invention.

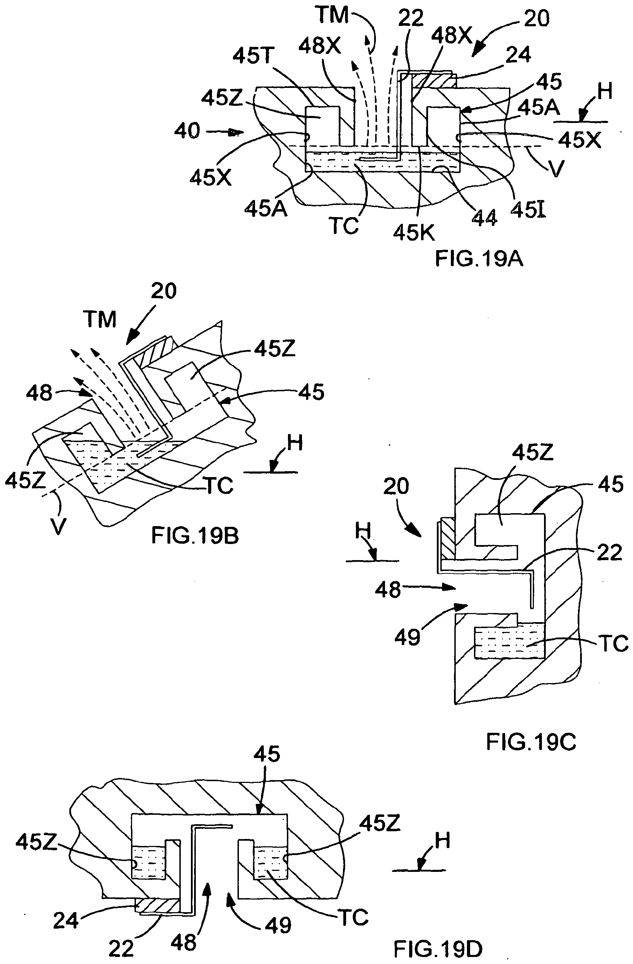

Figures 19A, 19B, 19C and 19D illustrate alternate views of a mist generator 20 according to the invention mounted in conjunction with an atomizing chamber 45 which has improved resistance to spilling of a treatment composition TC consequent to reorientation, e.g., tilting, or inversion of the atomizing chamber 45 of the device. For the sake of clarity, a fluid conduit 60 has been omitted from the figures but may be present at either as integral part of the first to body part 40 as previously depicted, or it may be a discrete separate element, e.g. as a pipe or tube for injecting the treatment composition TC into the interior of the atomizing chamber 45, although not shown in this figure. The atomizing chamber 45 has a base 44, a generally perpendicular side wall 45A which can be either a single circular side wall or maybe a plurality of flat or paneled sidewalls, such as would be required for a noncircular atomizing chamber 45, e.g., a square or rectangular shaped atomizing chamber 45. The side wall 45A and terminates at a top 45T, and extends from the side wall 45A to an inner sidewall 45I which is generally perpendicular to the top 45T and extends downwardly or inwardly towards the base 44 therefrom, until the inner sidewall 45I terminates at a inner sidewall base 45K. The inner sidewall 451 may be a single, circular sidewall or maybe a plurality of flat wall sections or panels depending from the top 45T and extending downwardly or inwardly towards the base 45 and terminating at the inner sidewall base 45K. the space defined between the inner sidewall 451, the top 45T and the sidewall 45A defines a chamber adapt its to contain the treatment composition TC when the atomizing chamber 45 and/or the device or oriented at positions respective to the horizontal, indicated by line segment "H", other than as shown on Fig. 19A. The inner sidewall base 45K is preferably, generally parallel to the base 44 and advantageously defines the bottom of an opening bore section 49, which extends and provides for a passage permitting for the transit of atomized particles of the treatment composition, to pass from within that the interior of the atomizing chamber 45, and outwardly through the opening 48. As is also visible, the mist generator 20 is present, with the piezoelectric element 24 and the depending L-shaped vibrating element 22 mounted such that the portion of the vibrating plate 22 having passages or microperforations as discussed with reference to Figs. 1, 2, 2A, 2B and 2C is in contact with the fluid treatment composition TC. Thus, when the mist generator 20 is caused to operate, the vibrating plate 22 forms a mist of the treatment composition TM which exits upwardly through the opening bore section 49 and outwardly from the opening 48. As is also visible from the figure, the maximum transverse dimension of the opening bore section 49, which in this embodiment is coincident with the dimensions of the opening 48, is determined as the distance between points 48X, which is lesser than the maximum transverse dimension of the atomizing chamber 45, which is determined as the distance between points 45X.
Figures 19B, 19C depict tilted orientations of the atomizing chamber 45 containing a quantity of the treatment composition TC, while Figure 19D depicts an inverted atomizing chamber 45 containing a quantity of the treatment composition TC. As is visible from each of these figures, the quantity of the treatment composition TC is retained within the confines of the atomizing chamber 45, particularly at least partially in chamber 45Z defined by the inner sidewall base 45K, the space defined between the inner sidewall 451, the top 45T and the sidewall 45A. Such is also depicted by the region between dotted line "V" and the top 45T. In the embodiment according to Fig. 19D were in the atomizing chamber 45 is inverted with respect to the horizontal, line segment "H", the quantity of the treatment composition TC is contained within the chamber 45Z. As can be seen from these four figures, the embodiment disclosed provides certain technical advantages. A first advantage is that is particular difficult to tilt or reorient the atomizing chamber such that actual spillage of the fluid treatment composition will occur, even upon total inversion of the atomizing chamber 45. A second advantage is that upon the selected placement of the vibrating plate 22 within the atomizing chamber 45 a useful degree of controlled operation responsive to the orientation of the atomizing chamber 45 can be established. For example, when the atomizing chamber 45 is inverted, as depicted on Fig. 19D, the mist generator 20 may operate, but will not generate a mist of the treatment composition TM. When inclined at a steep angle, such as 90° from the horizontal, as depicted on Fig. 19C, the mist generator 20 will also not operate. However, when inclined at a lesser angle with respect to the horizontal as depicted on Fig. 19B, the mist generator 20 will continue to operate and generate a mist TM of the treatment composition. Thus, by an appropriate configuration of the atomizing chamber 45, and the mist generator 20, relative to the overall design of the device, a useful degree of control of mist generation responsive to the orientation of the device can be achieved.
   A further embodiment of an atomizing chamber 45 and a mist generator 20 of a simplified construction not covered by the claims, but offering a somewhat lesser degree of resistance to spilling of a treatment composition TC consequent to reorientation, e.g., tilting or inversion, of the atomizing chamber 45 of the device is illustrated in Figures 20A, 20B, and 20C. Figure 20A depicts the atomizing chamber 45 in an orientation to be considered "level" with the horizontal, represented by reference line "H", while figures 20B and 20C depict the atomizing chamber 45 in an orientation can be considered as "tilted" with respect to the horizontal. As visible thereon, the atomizing chamber 45 is defined by a base 44 having an upper lead directing sidewall or sidewall 45, extending up to a top 45T which extends inwardly towards an opening 48. The atomizing chamber 45 has a maximum transverse dimension, here the distance between opposing points 45X, and the opening 48 as a maximum transverse dimension, here the distance between opposing points 48X, which distance is lesser of the two. Also the height of the sidewall or sidewall 45 is less than that of the maximum transverse dimension of the opening however, the maximum transverse direction of the atomizing chamber 45 is preferably at least as great as, or greater than the distance between opposing points 48X. Similarly to the embodiment depicted on Figures 19A - 19D, and mist generator 20 including a piezoelectric device 24 and an L-shaped vibrating plate is mounted with respect to the atomizing chamber 45 such that a portion of the vibrating plate 22 is in contact with the treatment composition TC present within the interior of the atomizing chamber 45. As is seen from each of Figs. 20A, 20B, and 20C the mist generator 20 will continue to operate to deliver a mist of the treatment composition TM when oriented "level" with the horizontal, or inclined with respect to the horizontal. The instant embodiment however, may allow for the escape of a fluid treatment composition TC via the opening 48 if the atomizing chamber 45 is further inclined or inverted with respect to the horizontal.
   While not disclosed in prior Figures 18A - 18C, 19A-19D or 20A-20C, it is to be understood that a mist generator 20 such as disclosed and discussed with reference to Figs. 1, 2, 2A, 2B and 2C may be used in place of the depicted mist generators 20 disclosed on these figures. Mist generators 20 according to Figs. 1, 2, 2A, 2B and 2C may be suitably placed within the interior of the atomizing chamber 35, preferably adjacent with the base 44 thereof. The configuration of the atomizing chamber disclosed in prior Figures 18A - 18C, 19A- 19D or 20A-20C or contemplated to provide similar resistance to spilling of a treatment composition TC consequent to reorientation, e.g., tilting, or inversion of the atomizing chamber 45 of the device.

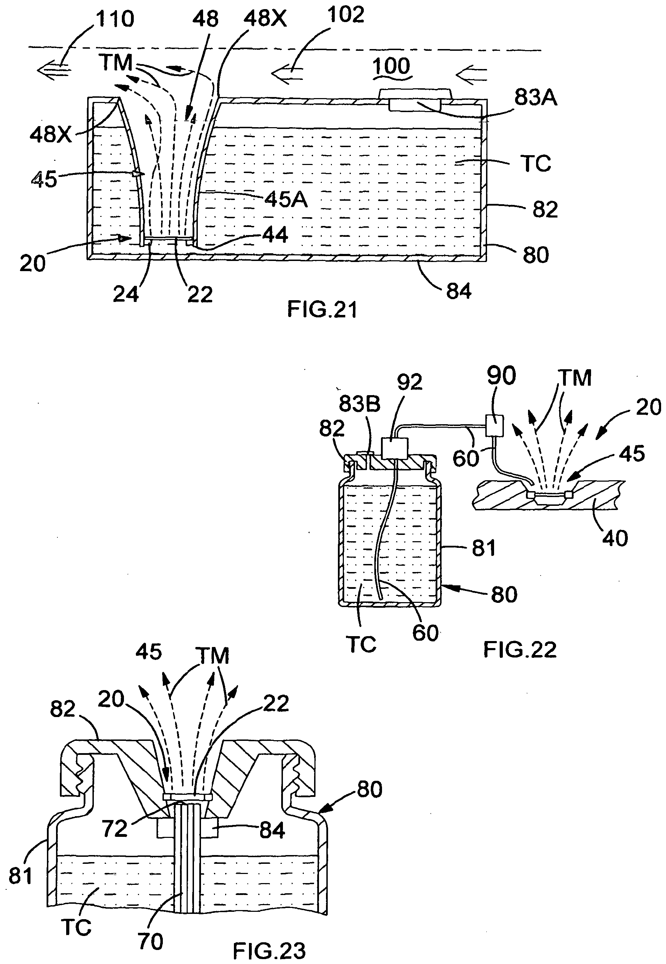

Fig. 21 an embodiment a mist generator 20 and an atomizing chamber 45 which is integrally formed within a reservoir 80, here in the form of a rectangular vessel 82 which contains within its interior a quantity of the treatment composition TC. The disclosed embodiments can be refilled by removing a replaceable plug element 83A supplying a quantity of the fluid treatment composition TC to the interior of the vessel 82 when required. The atomizing chamber 45 is integrally formed as part of the vessel 82. In the depicted embodiment, the atomizing chamber 45 comprises a base 45 which is near to or adjacent to the bottom 84 of the vessel 82. An outwardly tapering, or horn shaped sidewall 45A extends upwardly from the base 44 where it terminates at an opening 48 coincident with a top 85 of the vessel 82. A mist generator 20 means comprising a vibrating plate 22 and a piezoelectric element 24, e.g, as depicted in Figs. 1, 2, 2A, 2B and 2C is mounted transverse to the base 44. The dimensions of the passages or microperforations are preferably such that they are sufficiently small such that when the vibrating plate 22 is not activated and does not vibrate, but is at rest or in a static condition, the surface tension of the treatment composition TC is such that it does not flow through the these passages or microperforations. Thus, the static vibrating plate 22 acts as a valve for controlling the flow of the treatment composition. However, when the mist generator 20 operates, a mist of the treatment composition TM is formed by the vibrating plate 22 and passes upward through the atomizing chamber 45 and past the opening 48 where it is entrained by a flowing gas 100, preferably air, moving through the airflow conduit 100. The gas entraining the atomized treatment composition is depicted by arrow 110, which also represents the treatment mist. In the embodiment depicted, as the static vibrating plate 22 acts as a valve controlling the passage of the treatment composition TC from the reservoir 80, it is expected that the embodiment can it be used in virtually any position with little or no risk as to unintended spillage of fluid treatment composition TC from the device. Furthermore, the depicted embodiment is expected to permit operation of the mist generator 20 in any orientation as long as a quantity of the treatment composition TC is in contact with the vibrating plate 22.
Fig. 22 illustrates a further alternative embodiment of elements of a device similar to the invention. A reservoir 80 is provided, a hollow container 81 containing a quantity of the treatment composition TC in a fluid form, preferably in a liquid form, is attached to a removably affixed 82 cap. The cap has passing therethrough a capillary means a fluid conduit 60, here a flexible tube and fluid communication with a controllable pump 92 which is in communication with the controller means (not shown) and a suitable power supply source (not shown). The cap 82 also includes a venting valve 83B to permit for the entry of ambient air while the treatment composition TC is pumped from out of the reservoir 80. The fluid conduit 60 continues to a fluid control means 90 also in communication with the controller means and a power supply source which may be used to ensure that an optimal supply of the fluid treatment composition TC is transmitted to the atomizing chamber 45 to ensure desired operation of the mist generator 20. As visible on the figure, the fluid conduit 60 is separate from the first part 40 and supplies a controlled amount of the treatment composition TC responses to appropriate signal or control input from the controller means, to the mist generator 20. The mist generator 20 in the figure is similar to that depicted in Fig. 14, although it is understood that any other embodiment of a mist generator 20 may be interchangeably used.
Figure 23 depicts a further environment of elements of the device of the invention, which shows a partial view a reservoir 80 here a hollow container 81 closed by a removable cap 82. Integral to the 82 is depicted in cross-section an atomizing chamber 45 containing to a mist generator 20, e.g., in accordance with the embodiments of Fig. 1 or Fig. 2. Passing through a portion of the cap supported by a mounting ring 86 is a capillary means 70 whose terminal end 72 is beneath the vibrating plate 22, such that when then the mist generator 20 is operated, the treatment composition present at the terminal end 72 is pumped through the vibrating plate, and atomized to form a treatment mist TM. While not shown, suitable connections, e.g., wires, to the controller means and a power supply means may be provided.

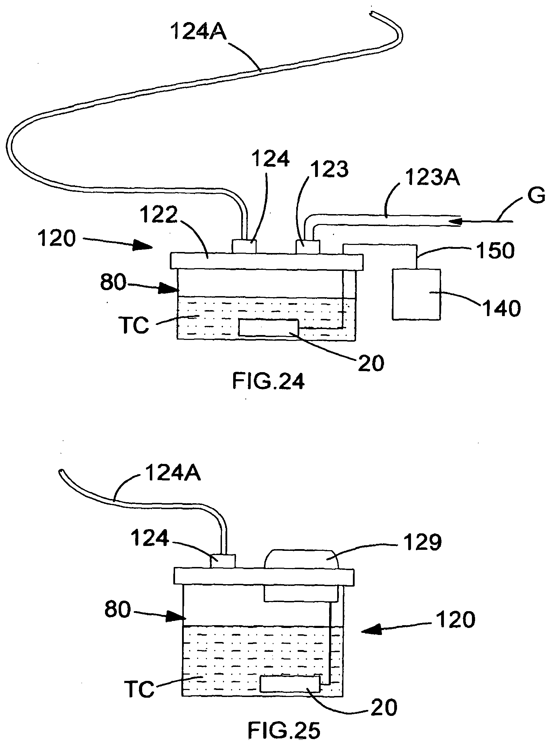

Figure 24 illustrates one embodiment of a part of a device 1 according to the present invention. The device 1 includes a first assembly 120 which includes a quantity of fluid treatment composition TC within a reservoir 80, a mist generator 20 submerged within the treatment composition TC which is attached to a controller means 140 by means of an intermediate wire or wires at 150, over which are also transmitted the power required to drive the mist generator 20. The first assembly 120 is openable via a top cover 122, which has passing therethrough two connector ports, an airflow inlet connector port 123 and a mist output connector port 124. While not depicted in the figure, but represented to by the arrow labeled "G" is an airflow generator means which provides a stream of a gas, preferably air via the airflow tube 123A which generates an elevated pressure within the interior of the vessel 80. The treatment composition in the form of a mist TM present within the vessel 80 is forced out via the mist tube 124A which directs it to a flow directing nozzle 162 at its a distal end 161 from which the mist of the treatment composition TM emanates which can be conveniently used to deliver a quantity of the mist of the treatment composition TM to a desired location.
Fig. 25 illustrates an alternative embodiment of a first assembly 120, which is a self-contained, in that the controller means, power supply source, and airflow generator are contained in the housing 129 forming a part of the first assembly 120, for example, a battery powered blower or fan may be used in providing sufficient pressure within the interior of the reservoir 80 so to cause the flow of the mist of the treatment composition through the mist tube 124A. Such a self-contained first assembly provides for a more integrated device 1 according to the invention.

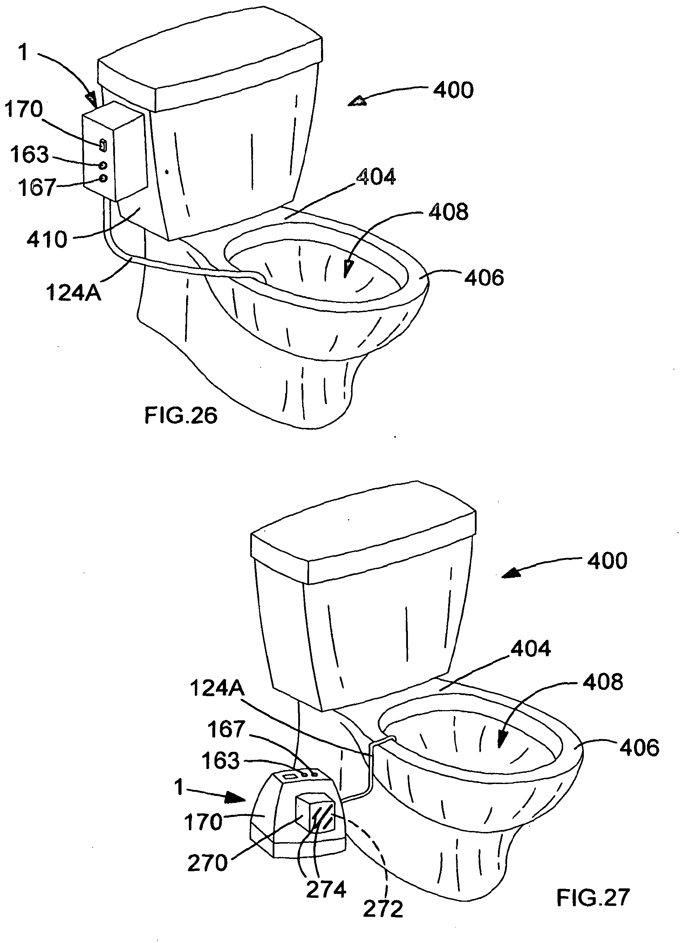

Figure 26 depicts in a perspective view an embodiment of a device 1 according to the invention mounted upon a toilet 400, more particularly a sidewall 410 forming part of the toilet cistern or toilet tank 410. The device may be removably mounted, or may be permanently mounted to a part of the toilet 400. Extending downwardly and away from the device 1 is provided a mist tube 124A which functions to deliver the mist of the treatment composition TM generated within the device 1 downwardly, to a flow directing nozzle (not shown) within the interior 408 of the toilet bowl 404. As seen in the figure, a part of the mist tube 124A traverses a part of the toilet bowl rim 406, and while not shown in the figure is to be understood that a conventional toilet seat (not shown) may be placed above the mist tube 124A. Further visible on the figure is a control button 163 and extending through a part of the housing 170, and as an indicator means 167, a plurality of light emitting diodes which provide information regarding the status of the device 1. While not depicted in the figure, is nonetheless to be understood that to a portion of the housing 170 may be removed, or dislocated in order to provide access to the interior of the device 1, such as for example, to replenish the quantity of the fluid treatment composition contained within a reservoir within the confines of the housing 170 either directly or via the use of a replaceable refill vessel or cartridge, as well as to change one or more electrical batteries which function as the power supply for the device 170. When operational, the device 1 delivers an airborne mist TM of the treatment composition to the interior 408 of the toilet bowl 404, in a sufficient amount in order to provide a desired technical benefit to the treated surfaces, particularly be treated in interior surfaces of the toilet bowl 404.
Figure 27 depicts in a perspective view a further embodiment of a device 1 according to the invention similar in several regards to the prior figure but, differing in and at the device 1 is positioned upon an adjacent flooring surface in the proximity of the toilet 400, and further wherein mounted on the exterior of the device 1 is an air-treatment means 270. Extending upwardly and away from the device is a mist tube 124 which operates to deliver a mist of the treatment composition and away from the device 1 is provided a mist tube 124A which functions to deliver the mist of the treatment composition TM generated within the device 1 downwardly, to a flow directing nozzle (not shown) within the interior 408 of the toilet bowl 404. As seen in the figure, a part of the mist tube 124A traverses is a part of the toilet bowl rim 406, and while not shown in the figure is to be understood that a conventional toilet seat (not shown) may be placed above the mist tube 124A. The device 1 also includes a control button 163 extending through a part of the housing 170, and as indicator means 167 a plurality of light emitting diodes. Although not depicted in this figure, is to be understood that the interior of housing 170 is accessible to a user or consumer in order to replenish the quantity of the fluid treatment composition contained therein, either directly or by replacing a fluid reservoir 80 with a new quantity of the fluid treatment composition, as well as to change one or more electrical batteries within the device 1. The depicted embodiment of an air-treatment means 270 is used to provide a volatile material to the ambient environment of the device, which volatile material is supplied to the ambient environment independently of the mist generator means. In the current embodiment the air-treatment means 270 comprises a capsule 272 having a plurality of perforations 274 passing therethrough, containing a fibrous pad within its interior impregnated with a fragrance composition. The fragrance composition volatilizes within the capsule 272 exiting via the plurality of perforations 274 wherein the airspace in the proximity of the toilet 400.

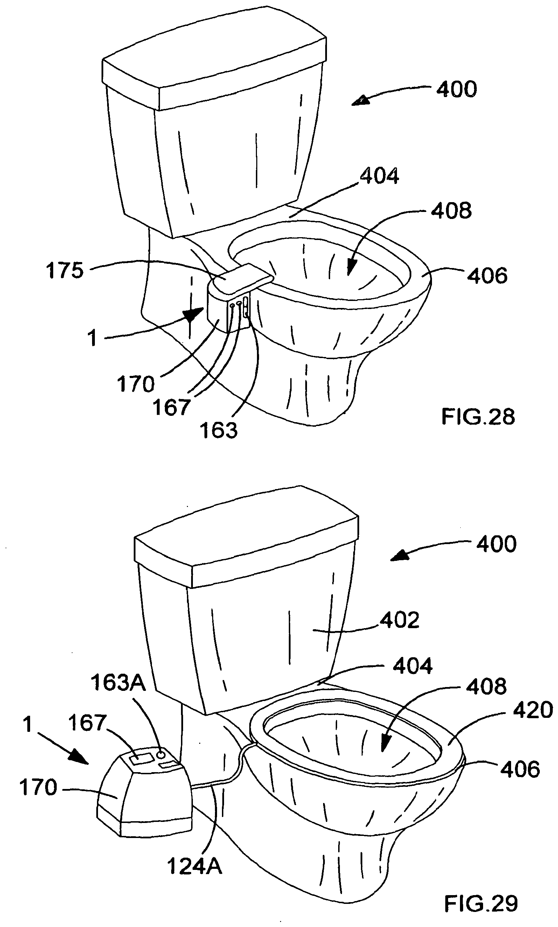

Figure 28 depicts in a perspective view a yet further embodiment of a device 1 according to the invention. In this embodiment, the device 1 is removably mountable upon a portion of the toilet bowl rim 406, wherein the housing 170 is connected to a an extended horn 175 which operates to both concurrently function as a hangar means for the device 1 and as a flow directing nozzle 162, through which the mist of the treatment composition TM generated by the device 1 is provided to the interior 408 of the toilet bowl 404. Similarly the device 1 includes control button 163 extending through a part of the housing 170, and as indicator means 167 a plurality of light emitting diodes. Also, the interior of housing 170 is accessible to a user or consumer in order to replenish the quantity of the fluid treatment composition contained therein, either directly or by replacing a fluid reservoir 80 with a new quantity of the fluid treatment composition, as well as to change one or more electrical batteries within the device 1.
Figure 29 illustrates in a perspective view and yet further embodiment of a device 1 according to the invention, similar in many respects to the embodiment disclosed with reference to Fig. 27. The device 1 includes in the place of a control button a sliding switch 163A, as well as a further includes as a status indicator means a light emitting diode (LED), and further also a small LCD or LED panel which displays visible symbols relevant to the operating status of the device 1. The slideable switch 163A is movable between two or more positions, and in its most simplest form operates only as an "on" and "off' switch, but preferably includes a least one or more intermediate settings. The one or more intermediate settings can be used to establish various operating parameters of the device 1, such as controlling the rate of delivery of the mist of the treatment composition, timer means to automatically engaged, and disengage operation of the device 1 at one or more preselected intervals of time and thereby providing for unattended operation of the device 1, or other operating parameters. Similarly, the status indicator means 167 may be other than light emitting diodes, and can be any visually discernible, audio discernible, tactile discernible indicators which provide information regarding the status of the device including the operating status of the device 1 to a user. For example, the status indicator means 167 may be a small LCD or LED panel which are properly displays symbols relevant to the operating status of the device, such as pictographs, icons, written words, numerical indicators, and the like. In the embodiment, and upwardly extending mist tube 124A is used to transfer a mist from the interior of the housing 170 of the device 1 to a flow directing seat manifold 420 positioned upon the rim 406. This flow directing seat manifold 420 operates to distribute the mist of the treatment composition being supplied, downwardly and inwardly into the toilet bowl 408, and functions as a flow directing nozzle.
   The operation of the flow directing seat manifold 420 is more clearly described with reference to the depiction of Fig. 30. In this cross-sectional view of a portion of the flow directing seat manifold 420 and a portion of the toilet bowl 404, the seat manifold 420 comprises a housing 422 defining and interior volume 423 through which the mist of the treatment composition TM may flow after being supplied by the mist tube (not shown). Advantageously, the interior volume 423 is substantially hollow although, one or more support structures such as internal posts, beams or buttresses may be present within the housing 422. One or more downwardly or inwardly directed orifices 424 are present within the lower rim 425 of the housing 422 which provide for passages through which the mist TM may pass and be directed in to the interior 408 of the toilet bowl 404. Although not depicted, it is to be understood that the dimensions and placement of the housing 422 of the seat manifold 420 may be dimensioned or configured to be placeable between a conventional toilet seat (not shown) and the portions of the rim 406 upon which the seat manifold 420 is placed.
   Advantageously, a sufficient supply of the mist TM is provided to the interior toilet bowl 408 so that it is essentially flooded by a quantity of the airborne mist TM. Advantageously, it has also been discovered in fact that typically, the temperature of the toilet bowl 408 may be at least slightly cooler than its surrounding environment, or due to the immediate proximity of a layer of water OW typically present in the sump or outlet 409 of the toilet bowl, the locally increased humidity within the interior 408 of the toilet bowl 404 may facilitate the rate of coalescence of the airborne particles present within the treatment mist TM such that they settle, in a generally uniform manner, upon the interior surfaces 408 including both inclined sidewalls 412, and often, as well as to a good degree the inner surfaces 414 of the underside of the rim 406 . This is due to the fact that prior to coalescing, the airborne treatment mist TM drifts within the interior volume of the toilet bowl 404.
Figure 31 illustrates one potential embodiment of a portion of the device 1. Here, in perspective view is illustrated in a clip hanger 450 which may be removably fitted onto portion of the rim 406 of a toilet bowl, which bears or is affixed to a further embodiment of a flow directing nozzle 162B. The clip hanger 450 advantageously includes a top plate part 451 from which depends both an inner plate part 452, and spaced away and apart therefrom an outer plate part 453. The clip hanger 450 is advantageously configured or proportioned to be positioned upon a portion of a toilet bowl rim 406, and conveniently the inner plate part 452 and outer plate part 453 are approximately or substantially parallel to each other, while both are approximately or substantially perpendicular to the top plate part 451. The flow directing nozzle 162B depicted here is generally a circular exit orifice present at a proximal end 162P of a segmented connector tube 162T which is borne or affixed to, or forms a part of the clip hanger 450. A distal end 162D of the segmented connector tube 162T is connected to a mist tube 124A which supplies a quantity of the mist of the treatment composition TM which passes through the segmented connector tube 162T, and exits in the nozzle 162B into the interior of the toilet bowl (not shown), which upon exiting disperses typically as a cone-like stream.
Figure 32 depicts another potential embodiment of a portion of the device 1, namely a further embodiment of a flow directing nozzle 162C which is otherwise similar in all other respects to the embodiment discussed with reference to Fig. 31. The flow directing nozzle 162C is circular or semicircular and includes a plurality of exit orifices 424 which aid in the distribution of the mist of the treatment composition exiting the flow directing nozzle 162C in a more disperse, and generally horizontal pattern such that the mist of the treatment composition TM is more evenly and laterally dispensed into the toilet bowl.

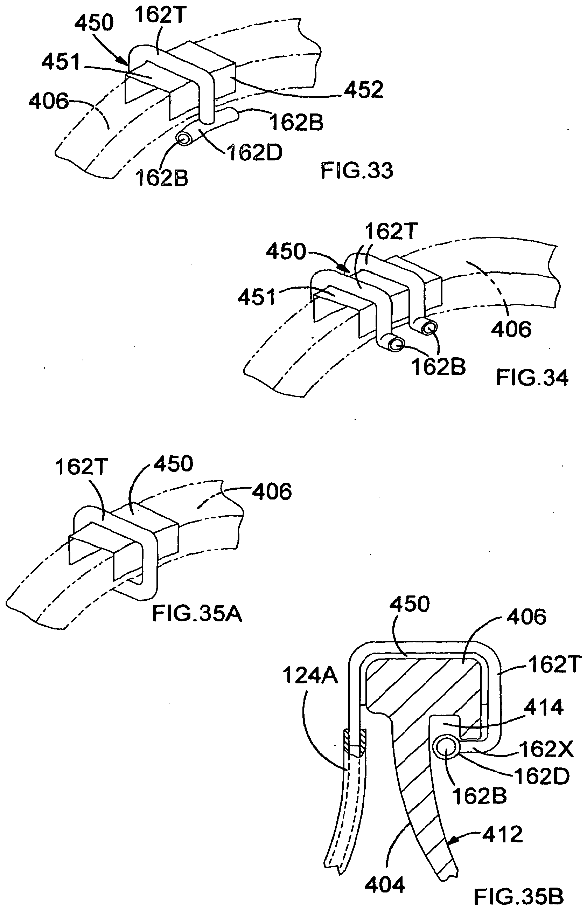

Figure 33 illustrates a still further potential embodiment of a portion of the device 1, here a yet further embodiment of a flow directing nozzle 162D, which comprises to generally circular exit orifices 162B at opposite ends of the "T-shaped" nozzle 162D. the mist of the treatment composition TM supplied to the nozzle 162D is simultaneously dispensed from the opposite ends there from the both out of the exit orifices 162B laterally and generally parallel to the rim 406. Where a sufficient velocity of the mist of the treatment composition TM exists, the said mist extends in a "curtain like" fashion along of the sides of the interior of the toilet bowl.
Figure 34 depicts in a perspective view of a yet further potential embodiment of a portion of the device 1, here, a pair of flow directing nozzles 162B, substantially as described with reference to Fig. 31. The provision of a pair of separate flow directing nozzles 162B permits for the simultaneous, or sequential dispensing of a mist of a treatment composition TM, alternately for the simultaneous or sequential dispensing of two different mists of two (or more) treatment compositions TM. It may be desired in certain embodiments, to provide two different treatment mists TM which are kept separate, and only come into contact with each other after exiting the flow directing nozzles 162B where they may chemically or physically react with one another after such contact.
Figures 35A and 35B depicts two views of a still further embodiment of a portion of the device 1, here a further embodiment of a "T-shaped" nozzle 162D, which is similar in many regards to that described with reference to Fig. 33. In the depicted embodiment, the distal end 162D of the segmented connector tube 162T is connected to a mist tube 124A which supplies a quantity of the mist of the treatment composition TM which passes through the segmented connector tube 162T, and through an extended section thereof, 162X, and then enters the flow directing nozzle 162D and thereafter exits via the two exit orifices 162B thereof. In this manner the mist of the treatment composition TM is dispensed in the inner surfaces 414 of the underside of the rim 406 of the toilet bowl 404. Again, where a sufficient velocity of the mist of the treatment composition TM exists, the said mist TM extends in a "curtain like" fashion on the underside of the rim 406 of the toilet bowl 404 before drifting downward along of the interior curved sides 412 of the toilet bowl 404.

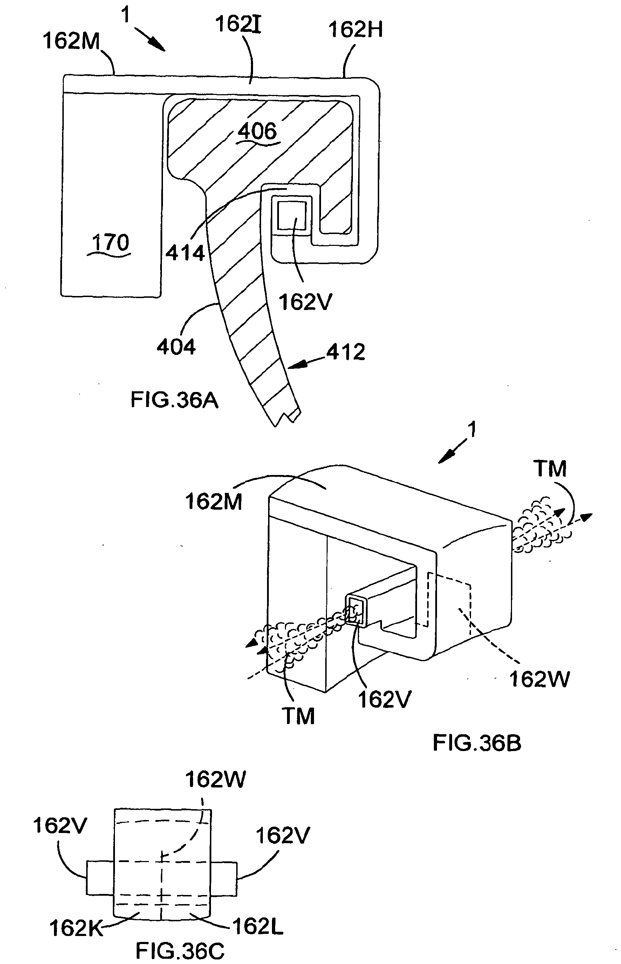

Figures 36A, 36B and 36C depict various view of a preferred embodiment of a device 1 according to the invention adapted to be installed and suspended upon a toilet bowl rim 414. A portion of the housing 170 of the device, or alternately a part extending therefrom is a flow directing nozzle assembly 162H which operates to both direct the flow of a mist of a treatment composition TM passing through its interior plenum 1621 and ultimately directing its dispensing through two, oppositely directed exit orifices 162V, as well as to function as a hanger means for suspending the device 1. Mist of the treatment composition TM passing from within the housing 170 enters the interior plenum 1621 had a proximal end 162M thereof, and flows in the direction of the exit orifices 162V, in a manner similar to that previously described with respect to the segmented connector tube 162T. The mist TM is divided into two separate volumes or streams by a dividing wall 162W (illustrated in dotted lines) which splits the plenum into two separate by humans downstream thereof, thereby ideally approximately equally splitting the quantity of the mist of treatment composition TM into two parts, each part of which separately exits one of the two oppositely directed exit orifices 162V, which are placed beneath the rim 406 of the toilet bowl 404. Thus in this manner the mist of the treatment composition TM is dispensed in the inner surfaces 414 of the underside of the rim 406 of the toilet bowl 404, and where sufficient volume and velocity of the treatment mist TM is present, the mist TM exiting in opposite directions and proximate to the inner surfaces 414 extends in a "curtain like" fashion along the interior curved sides 412 of the toilet bowl 404 of the toilet bowl 404.

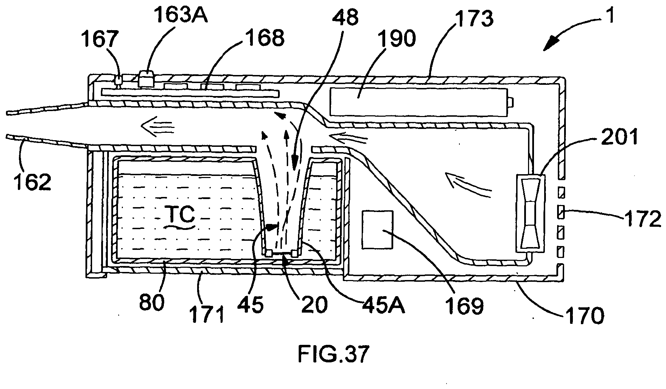

Figure 37 depicts an embodiment of a part of a device 1. As is seen in this cross sectional view, the housing 170 contains within its interior an embodiment a mist generator 20 and an atomizing chamber 45 which is integrally formed within a reservoir 80, here in the form of a rectangular vessel 82 which contains within its interior quantity of the treatment composition TC, similar in most respects to that described with reference to Fig. 21. The rectangular vessel 82 may be inserted within the housing 170 via the removable cover 171. The opening 48 of the atomizing chamber 45 opens within the airflow conduit 100, which at one end includes a radial electrical fan 201, and at the other end terminates in a mist tube 124A which is connected to a flow directing nozzle (not shown) but alternately, may directly terminate in a flow directing nozzle (not shown) which by way of non-limiting example may be one or more of those disclosed in any of the prior figures. An air intake grille 172 is also present in the housing 170 The controller means 168 controls the operating characteristics of the device 1, and in particular the operating parameters of the fan 201, and the mist generator 20. Power may be supplied to the device via one or more electrical batteries 190 which may be located beneath the cover part 173 of the housing 170. The device further includes orientation sensing means 169 for determining a physical orientation of the device, which for example, can be a level sensor, horizon sensor, accelerometer or any other device which can be used to establish the relative position of the device 1. with respect to the horizontal or horizon. The device also includes a transmitter means 71 and a receiver means 72 positioned transversely across the airflow conduit 100. The controller means may receive a signal input from the receiver 72 which relates to one or more conditions relevant to the operation of the device 1, e.g., the mass flow rate of the atomized treatment composition, and/or the particle size or particle size distribution of the atomized particles passing between the transmitter 71 and the receiver 72, and exiting the device 1. Such conditions may be represented by a suitable signal which is returned as feedback to the controller means which may then be used to transmit appropriate control signals and/or alter power being supplied to relevant elements of the device 1, e.g., the mist generator 20, controllable pump 92 or fan 201 so to modify the operating characteristics thereof so to return the operating parameters of the device to a state or one of several desired states of operation. The controller means 168 may also respond to signals received from an orientation sensing means in order to control the operating characteristics of the device 1, e.g., shutting off one or more parts of the device as an excessive degree of tilting is sensed, or if the device 1 is suddenly dropped or overturned. Other operative characteristics of the device including but not limited to blower rotational speed, as well as operative characteristics of the mist generator 20 can also be independently or simultaneously controlled by the controller means 168, which may include one or more electronic components containing a hardware circuit, or a logic processor, or central processing unit which may operates the device 1 responsive to a preset program, which can be stored in a volatile but preferably nonvolatile memory means present on the controller card. The controller means 168 may operate according to one preprogrammed mode of operation, or might alternately operate according to two or more preprogrammed modes of operation which can be selected by appropriate placement of the slideable switch 163A. Further, the operative status of the device can be indicated by the status indicator means 167.
   An alternate interior embodiment of a device is illustrated in the cross-sectional view of Figure 38. The depicted embodiment is similar in some respects to that of prior Fig. 37. In the present embodiment however, a self-contained blower motor 200 is included, which supplies airflow into the airflow conduit 100. The mist generator 20 is similar to the embodiment discussed with reference to Fig. 13. Fluid treatment composition TC is contained within the interior of the vessel 82, and the quantity of the treatment composition TC can be replenished via removal of the plug 83. He flew conduit 60 communicates from within the interior of the vessel 82, and a controllable pump 92 which is controlled by the controller means 168. A controllable pump 92 supplies a quantity of the fluid treatment composition when necessary to the mist generator 20. The mist generator 20 is similar to, and works similarly to the embodiment discussed with reference to Fig. 13. The operation of the mist generator 20 is also controlled by the controller means 168. The mist of the treatment composition TM is entrained in the airflow generated by a blower 200, and is directed outward from the device 1 via a mist tube 124A which is connected to a flow directing nozzle (not shown) but alternately, may directly terminate in a flow directing nozzle (not shown) which by way of non-limiting example may be one or more of those disclosed in any of the prior figures. Necessary electrical power is supplied to components of the device 1 via batteries 190. Necessary interconnections between electrically operated or operable components of the device are supplied via suitable signal and/or power transmission means, e.g., wires which are not shown in the figure for purposes of clarity. The device further includes orientation a pair of sensing means 169, 169 for determining a physical orientation of the device, which for example, can be a level sensor, horizon sensor, accelerometer or any other device which can be used to establish the relative position of the device 1 with respect to the horizontal or horizon. The pair of sensing means 169, 169 may be oriented perpendicular to each other within the device 1 such that signals indicative of "up or down tilting" of the device with respect to the horizon, e.g., in a first vertical reference plane traversing along the length of the airflow conduit 100, as well as signals indicative of "side to side tilting" of the device with respects to the horizon, e.g, and a second vertical reference plane perpendicular to the first reference plane traversing along the length of the airflow conduit 100. Such provides for improved signal inputs with regard to the position of the device 1. relative to its operating environment.
   A further, alternative interior environment of the device of Fig. 36 is depicted in the cross-sectional view of Figure 39. The depicted embodiment is similar in several respects to those of prior Figs. 37 and 38. In this embodiment, the airflow generator means comprises an aerosol canister 230 containing a propellant or a pressurized gas, having an actuator 231 held in a seal-tight relationship with a controlled valve means 232, responsive to an appropriate signal input from the controller means 168 is actuated to release a quantity of the propellant or pressurized gas from the aerosol canister 230 into the airflow conduit 100. Such a release may be periodic, or continuous during the operation of the device. The control valve means 32 may be any device which provides this operative function, and for example may be a simple solenoid which operates as a plunger to operate the actuator 231, or may be an electrically operated solenoid valve such as disclosed in one or more of patents US 7100889, US 6328279, US 5356111. The released gas from the aerosol canister 230 entrains the mist of the treatment composition TM generated by the mist generator 20. A controllable pump 92 supplies, via fluid conduits 60, quantities of treatment composition TM contained within the vessel 82 to the mist generator 20. The mist generator 20 is generally disclosed with reference to the embodiment discussed with reference to Fig. 13. The vessel 82, and the aerosol canister 230 be supplied as part of a refill cartridge 87 which is removable from, and replaceable into the interior of the housing 170 via a removable cover 171. A further, similar removable cover 171 may also be present to allow for the replacement of the one or more batteries 190 used to provide electrical power to components of the device 1. The device may also further include one or more sensing means 169 operatively connected to the controller means 168.
   A yet further, alternative interior environment of the device of Fig. 36 is depicted in the cross-sectional view of Figure 39A. The depicted embodiment is similar in several regards to those of prior Figs. 37, 38 and 39 and includes common features thereto. In the current embodiment, a portion of the reservoir 80 comprises a vertically disposed mist generator 20, which may be as those described with reference to Figs. 1, 2, or 2A, which is vertically disposed such that one side of the vibrating plate 22 is in direct contact with the quantity of the treatment composition TC present in the reservoir 80. The reservoir 80 may be refilled via a removeable and replaceable plug 83A. While operating, the vibrating plate 22 of the mist generator means 20 produces a treatment mist TM which enters the airflow conduit 100 wherein it is entrained by a flow of air being generated by a blower 200, such that the mist of treatment composition TM is directed outwards of the device 1 where it exits via the flow directing nozzle 162. The embodiment of the device 1 shown in the figure illustrates an embodiment wherein a pump for supplying the fluid treatment composition to the mist generator 20 is not needed.

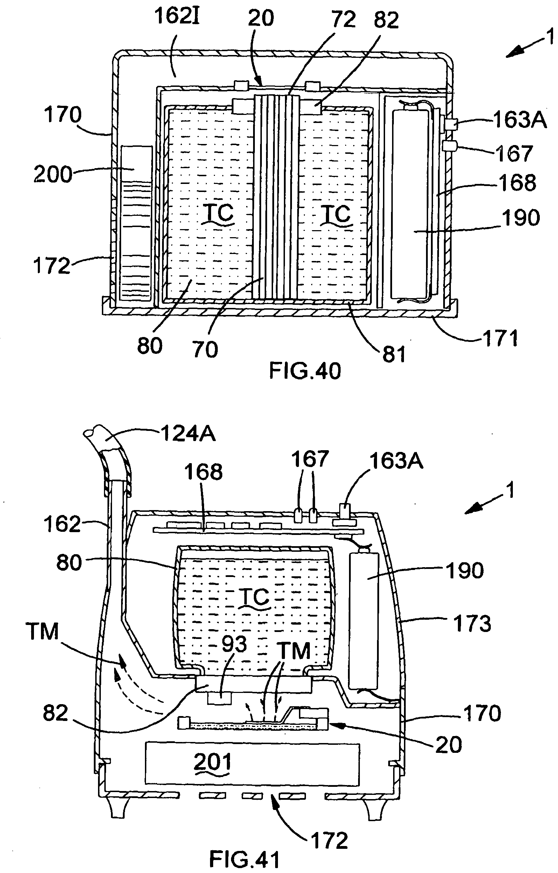

Figure 40 illustrates in cross-sectional view a further embodiment of a portion of a device 1. A shaped housing 170 having a removable and replaceable cover 171 contains within its interior as an airflow generator a blower 200, a power supply source 190, here one or more batteries, a controller means 220, including a slideable switch 163A as well as indicator means 167. A reservoir 80, here a container 81 containing a quantity of the treatment composition in a fluid form TC and a capillary means 70, here a porous fibrous element, is fitted such that the terminal end 72 thereof is proximate to a mist generator 20. The reservoir is removeable from within, and insertable into the housing 170. The housing 170 extends into a flow directing nozzle assembly 162H such as disclosed on Figs. 36A, 36B and 36C such that the mist of treatment composition TM formed by the mist generator 20 is induced to pass into and though the plenum 1621 by virtue of the stream of air generated by the blower 200. In operation, a user (human) actuates the controller means 220, e.g., by appropriately setting the switch 163A which acts to energize and operate the mist generator 20 and to also drive the blower 200 thereby causing airflow to traverse the mist generator 20 which volatilizes the fluid treatment composition TM being supplied via a capillary means 70, which also directs the mist of treatment composition through the plenum 1621 and ultimately to deliver the mist TM to a surface, especially a lavatory surface, e.g. one or more surfaces of a lavatory appliance.
Figure 41 illustrates a cross sectional view of a still further embodiment of part of a device 1, which is placeable in a stationary position near a lavatory appliance, e.g., on the floor near a lavatory appliance, and operated to generate and provide a mist of the treatment composition TM which may delivered to a remotely positioned flow directing valve (not shown) via a connecting mist tube 124A. A housing 170 including a removable cover part 173 which provides access for replacement of a vessel 80 containing a quantity of a fluid treatment composition TC. The vessel 80 is inverted, and includes a cap 82 which incorporates within its construction a controllable drip valve 93 which is responsive to appropriate signals from the controller means 168. The drip valve 93 provides a measured release of the fluid treatment composition TC into the mist generator 20 which is placed directly beneath such that, fluid treatment composition TC impinging thereon is atomized and forms a mist TM of the treatment composition TM. The mist generator depicted is similar in most respects to that described with reference to Fig. 13. Beneath the mist generator 20 is positioned a radial electrical fan 201 which provides airflow within the housing 170. Air enters the housing 170 via the grille 172, is accelerated by the fan 201, and the increased pressure forces the mist of the treatment composition TM out from the interior of the housing 170 via the exit orifice 162 which is connected to a mist tube 124A which transports the mist TM to a flow directing nozzle (not shown). The device 1 may also include within the housing 170 a power supply source such as one or more batteries 190 although such may be substituted by an alternative power source, e.g., a wired connection to electrical mains, or to an electrical transformer.

## Claims

1. A device for the treatment of a lavatory appliance which device generates a mist of a treatment composition which imparts a technical benefit to surfaces of the lavatory appliance which come into contact with the said aerosolized treatment composition, the device comprising: a mist generator means(20) including an atomizing chamber(45), a control circuit for operating the mist generator means(20), a reservoir for the fluid product to be aerosolized, a means for supplying the mist generating means with the fluid product, a housing(129,170), and at least one flow directing nozzle(162), flow directing implement or flow directing orifice adapted to direct the flow of a mist generated by the mist generating means(20) out from the housing(129,170) and towards part of a lavatory appliance, wherein that the atomizing chamber(45) has a base(44), a generally perpendicular side wall(45A)which terminates at a top, the top extends from the side wall to an inner sidewall which is generally perpendicular to the top and extends downwardly or inwardly towards the base(44) therefrom, until the inner sidewall(45L) terminates at an inner sidewall base(45K), **characterised in that** the mist has a bi-modal distribution of fine particles, with a first portion having a particle size of 1-8 microns and a second portion having a particle size of 10-40 microns,

2. A device according to claim 1, wherein the mist generating means(20) comprises a vibrating member(22) and a piezoelectric actuator(24).

3. A device according to claim 1 or 2, wherein the mist generating means(20) comprises a metal or ceramic plate as the vibrating member(22) which is attached to a piezoelectric material(24).

4. A device according to claim 1 wherein the reservoir is refill unit.

5. A device according to claim 1 wherein the reservoir of the device is a refill unit, the device includes a piezoelectric actuator(24), the refill unit includes a vibrating member(22), wherein the device and the refill unit are configured such that when the refill unit is properly installed in the device the piezoelectric actuator(24) and the vibrating member(22) interact to form the mist generator means(20).

6. A method for the treatment of surfaces associated with a lavatory appliance which method comprises the step of: providing a device according to any of claims 1 - 5, and operating the device to generate a mist of a treatment composition, which treatment composition contacts one or more surfaces of the lavatory appliance and provides a technical benefit thereto.

7. The use of a device according to any of claims 1 - 5 for the treatment of a lavatory appliance.

8. A method for the delivery of an air treatment composition to an airspace in the proximity of a lavatory appliance, which method comprises the step of: providing a device according to any of claims 1 - 5 which generates a mist of a treatment composition, which treatment composition contacts said airspace within the proximity of the lavatory appliance and provides a technical benefit thereto.

## Patentansprüche

1. Vorrichtung für die Behandlung einer Toiletteneinrichtung, welche Vorrichtung einen Nebel einer Behandlungszusammensetzung erzeugt, die Oberflächen der Toiletteneinrichtung, die mit der aerosolisierten Behandlungszusammensetzung in Kontakt kommen, einen technischen Vorteil verleiht, wobei die Vorrichtung umfasst: eine Nebelerzeugungseinrichtung (20), umfassend eine Zerstäuberkammer (45), eine Steuerschaltung zum Betreiben der Nebelerzeugungseinrichtung (20), ein Reservoir für das zu aerosolisierende Fluidprodukts, eine Einrichtung zum Versorgen der Nebelerzeugungseinrichtung mit dem Fluidprodukt, ein Gehäuse (129, 170) und wenigstens eine flusslenkende Düse (162), flussrichtende Einrichtung oder flusslenkende Öffnung, die dafür ausgelegt ist, den Fluss eines von der Nebelerzeugungseinrichtung (20) erzeugten Nebels aus dem Gehäuse (129, 170) und zu einem Teil einer Toiletteneinrichtung zu lenken, wobei die Zerstäuberkammer (45) eine Basis (44) und eine allgemein senkrechte Seitenwand (45A), die an einer Oberseite endet, umfasst, wobei die Oberseite von der Seitenwand zu einer inneren Seitenwand verläuft, die allgemein senkrecht zu der Oberseite ist und davon nach unten oder innen zu der Basis (44) verläuft, bis die innere Innenwand (45L) an einer inneren Seitenwandbasis (45K) endet, **dadurch gekennzeichnet, dass** der Nebel eine bimodale Verteilung von feinen Partikeln aufweist, wobei ein erster Teil eine Partikelgröße von 1-8 Mikrometer aufweist und ein zweiter Teil eine Partikelgröße von 10-40 Mikrometer aufweist.

2. Vorrichtung gemäß Anspruch 1, wobei die Nebelerzeugungseinrichtung (20) ein Vibrationselement (22) und einen piezoelektrischen Aktor (24) umfasst.

3. Vorrichtung gemäß Anspruch 1 oder 2, wobei die Nebelerzeugungseinrichtung (20) eine Metall- oder Keramikplatte, die an einem piezolektrischen Material (24) befestigt ist, als das Vibrationselement (22) umfasst.

4. Vorrichtung gemäß Anspruch 1, wobei das Reservoir eine Nachfülleinheit ist.

5. Vorrichtung gemäß Anspruch 1, wobei das Reservoir der Vorrichtung eine Nachfülleinheit ist, die Vorrichtung einen piezoelektrischen Aktor (24) umfasst, die Nachfülleinheit ein Vibrationselement (22) umfasst, wobei die Vorrichtung und die Nachfülleinheit so gestaltet sind, dass, wenn die Nachfülleinheit geeignet in die Vorrichtung eingesetzt ist, der piezoelektrische Aktor (24) und das Vibrationselement (22) wechselwirken, um die Nebelerzeugungseinrichtung (20) zu bilden.

6. Verfahren zur Behandlung von Oberflächen, die mit einer Toiletteneinrichtung verbunden sind, wobei das Verfahren den Schritt umfasst: Bereitstellen einer Vorrichtung gemäß einem der Ansprüche 1-5 und Betreiben der Vorrichtung, um einen Nebel einer Behandlungszusammensetzung zu erzeugen, welche Behandlungszusammensetzung mit einer oder mehreren Oberflächen der Toilettenvorrichtung in Kontakt kommt und ihnen einen technischen Vorteil verleiht.

7. Verwendung einer Vorrichtung gemäß einem der Ansprüche 1-5 für die Behandlung einer Toiletteneinrichtung.

8. Verfahren zum Abgeben einer Luftbehandlungszusammensetzung an einen Luftraum in der Nähe einer Toiletteneinrichtung, wobei das Verfahren den Schritt umfasst: Bereitstellen einer Vorrichtung gemäß einem der Ansprüche 1-5, die einen Nebel einer Behandlungszusammensetzung erzeugt, welche Behandlungszusammensetzung mit dem Luftraum in der Nähe der Toiletteneinrichtung in Kontakt kommt und ihm einen technischen Vorteil verleiht.

## Revendications

1. Dispositif pour le traitement d'un appareil de toilettes, lequel appareil produit un brouillard d'une composition de traitement qui confère un avantage technique aux surfaces de l'appareil de toilettes qui viennent en contact avec ladite composition de traitement en aérosol, le dispositif comprenant : un moyen (20) de production de brouillard comprenant une chambre d'atomisation (45), un circuit de commande pour faire fonctionner le moyen (20) de production de brouillard, un réservoir pour le produit fluide à vaporiser, un moyen pour alimenter le moyen de production de brouillard en produit fluide, un boîtier (129, 170), et au moins une buse (162) directrice d'écoulement, un outil directeur d'écoulement ou un orifice directeur d'écoulement apte à diriger l'écoulement du brouillard produit par le moyen (20) de production de brouillard hors du boîtier (129, 170) et vers une partie de l'appareil de toilettes,
dans lequel la chambre d'atomisation (45) comporte une base (44), une paroi latérale (45A) dans l'ensemble perpendiculaire qui se termine à un dessus, le dessus s'étend de la paroi latérale à une paroi latérale intérieure qui est dans l'ensemble perpendiculaire au sommet et s'étend de celui-ci vers le bas ou vers l'intérieur en direction de la base (44), jusqu'à ce que la paroi latérale intérieure (45L) se termine au niveau d'une base (45K) de paroi latérale intérieure, **caractérisé en ce que** le brouillard a une distribution bimodale de particules fines, avec une première partie présentant une taille de particule de 1 à 8 microns et une seconde partie présentant une taille de particule de 10 à 40 microns.

2. Dispositif selon la revendication 1, dans lequel le moyen (20) de production de brouillard comprend un élément vibrant (22) et un actionneur piézoélectrique (24).

3. Dispositif selon la revendication 1 ou 2, dans lequel le moyen (20) de production de brouillard comprend en tant qu'élément vibrant (22) une plaque en métal ou en céramique qui est fixée à un matériau piézoélectrique (24).

4. Dispositif selon la revendication 1, dans lequel le réservoir est une unité de recharge.

5. Dispositif selon la revendication 1, dans lequel le réservoir du dispositif est une unité de recharge, le dispositif comprend un actionneur piézoélectrique (24), l'unité de recharge comprend un élément vibrant (22), dans lequel le dispositif et l'unité de recharge sont configurés de telle sorte que, quand l'unité de recharge est correctement installée dans le dispositif, l'actionneur piézoélectrique (24) et l'élément vibrant (22) interagissent pour constituer le moyen (20) de production de brouillard.

6. Procédé pour le traitement de surfaces associées à un appareil de toilettes, lequel procédé comprend les étapes consistant à : faire appel à un dispositif selon l'une quelconque des revendications 1 à 5 et faire fonctionner le dispositif pour produire un brouillard d'une composition de traitement, laquelle composition de traitement entre en contact avec une ou plusieurs surfaces de l'appareil de toilettes et leur offre un avantage technique.

7. Utilisation d'un dispositif selon l'une quelconque des revendications 1 à 5 pour le traitement d'un appareil de toilettes.

8. Procédé pour libérer une composition de traitement d'air dans un volume à proximité d'un appareil de toilettes, lequel procédé comprend l'étape consistant à : faire appel à un dispositif selon l'une quelconque des revendications 1 à 5 qui produit un brouillard d'une composition de traitement, laquelle composition de traitement entre en contact avec ledit volume d'air à proximité de l'appareil de toilettes et lui offre un avantage technique.
